# EUROPEAN PATENT SPECIFICATION

(11) **EP 0 981 620 B1**
(45) Date of publication and mention of the grant of the patent: **25.02.2004**
(21) Application number: 98920645.3
(22) Date of filing: 07.05.1998
(51) Int. Cl.: C12N 15/19, C12N 5/10, C07K 14/475, A61K 38/12

(54) **HGF POLYPEPTIDES AND THEIR USE IN THERAPY**
HGF POLYPEPTIDE UND DEREN THERAPEUTISCHE VERWENDUNGEN
POLYPEPTIDES DU FACTEUR DE CROISSANCE DE LA CELLULE HEPATIQUE ET LEUR UTILISATION DANS DES TRAITEMENTS

(30) Priority: 10.05.1997 GB 9709453
(43) Date of publication of application: 01.03.2000
(73) Proprietor: Medical Research Council, London W1B 1AL (GB)
(72) Inventor: GHERARDI, Ermanno, Cambridge CB1 4RX (GB); BIRCHMEIER, Walter, D-16341 Schwanebeck (DE); HARTMANN, Guido, Cambridge CB1 3SE (GB)
(74) Representative: Miles, John Stephen
(86) International application number: PCT/GB1998/001318
(87) International publication number: WO 1998/051798

(56) References cited:
- EP-A- 0 757 994
- WO-A-93/23541
- SAKATA H. ET AL.: "Heparin binding and oligomerization of Hepatocyte Growth Factor/Scatter Factor isoforms" J. BIOL. CHEM., vol. 272, no. 14, 4 April 1997, pages 9457-9463, XP002074885
- MIZUNO K. ET AL.: "Hairpin loop and second Kringle domain are essential sites for heparin binding and biological activity of Hepatocyte Growth Factor" J. BIOL. CHEM., vol. 269, no. 2, 14 January 1994, pages 1131-1136, XP002074886 cited in the application
- HARTMANN G. ET AL.: "Engineered mutants of HGF/SF with reduced binding to heparan sulphate proteoglycans, decreased clearance and enhanced activity in vivo." CURRENT BIOLOGY, vol. 8, no. 3, - 29 January 1998 pages 125-134, XP002074628

## Description

The present invention relates to polypeptides and their use in therapy and in particular to the use of mutant hepatocyte growth factors in therapy.

Hepatocyte growth factor (HGF), also known as scatter factor (SF) is a polypeptide growth factor which induces growth, movement and differentiation in target cells. HGF is synthesised as a single-chain precursor which is proteolytically cleaved to an active two-chain form. The active form of HGF is believed to mediate its effect by binding to a tyrosine kinase receptor (MET) encoded by the c-*met* oncogene.

Wild-type HGF has a number of potential therapeutic uses. However, wild-type HGF is rapidly eliminated from the plasma (t½ approximately 4.5 min). The clearance rate can be reduced somewhat by complexing HGF to heparin or other polyanions prior to administration.

Matsumoto *et al* (1991) *Biochem. Biophys. Res. Comm.* **181**, 691-699 describes that the deletion of kringle domains or the N-terminal hairpin structure in HGF results in marked decreases in related biological activities.

Miau *et al* (1996) *Biochem. Biophys. Res. Comm.* **223**, 487-491 purports to describe the identification of a novel variant HGF secreted by spleen-derived stromal cells.

Lokker *et al* (1994) *Prot. Eng.* **7**, 895-903 describes the mutational analysis and molecular modelling of the N-terminal kringle-containing domain of HGF and identifies amino acid side chains important for interaction with the c-Met receptor.

Mizuno *et al* (1994) *J. Biol. Chem.* **269**, 1131-1136 describes mutants in which the hairpin loop or a kringle domain have been deleted.

Schwall *et al* (1996) *J. Cell Biol.* **133**, 709-718 reports that heparin induces dimerization and confers proliferative activity onto the HGF antagonists NK1 and NK2 which are truncated variants of HGF.

Lokker *et al* (1992) *EMBO J.* **11**, 2503-2510 describes various mutants of HGF.

WO 92/05184 and WO 96/40914 relate to a truncated form of HGF which purportedly antagonises the activity of HGF.

WO 93/23541, US 5,547,856, US 5,316,921 and US 5,580,963 relate to HGF variants that are resistant to proteolytic cleavage.

US 5,464,815 describes a method for extending the plasma half-life of heparin-binding proteins.

WO 96/28475 describes HGF modified with polyethylene glycol.

The present invention describes HGF variants which have significantly reduced ability to bind heparin or heparan sulphate proteoglycan (HSPG) but which are still able to bind to the HGF receptor (MET). At least some of these variants have a longer circulatory half-life *in vivo and* a greater mitogenic activity than wild-type HGF when administered to rats. Thus, one object of the invention is to overcome the need to complex HGF with heparin-like compounds or other polyanions in order to improve pharmacokinetics and *in vivo* activity. The variant HGF molecules of the invention may activate the HGF receptor in substantially the same way as wild-type HGF or they may act as antagonists of wild-type HGF or they may act in some other way. In any case the variant HGF molecules are useful in medicine.

A first aspect of the invention provides a variant hepatocyte growth factor (HGF) which is substantially incapable of binding a heparan sulphate proteoglycan but which is capable of binding to the HGF receptor for use in medicine.

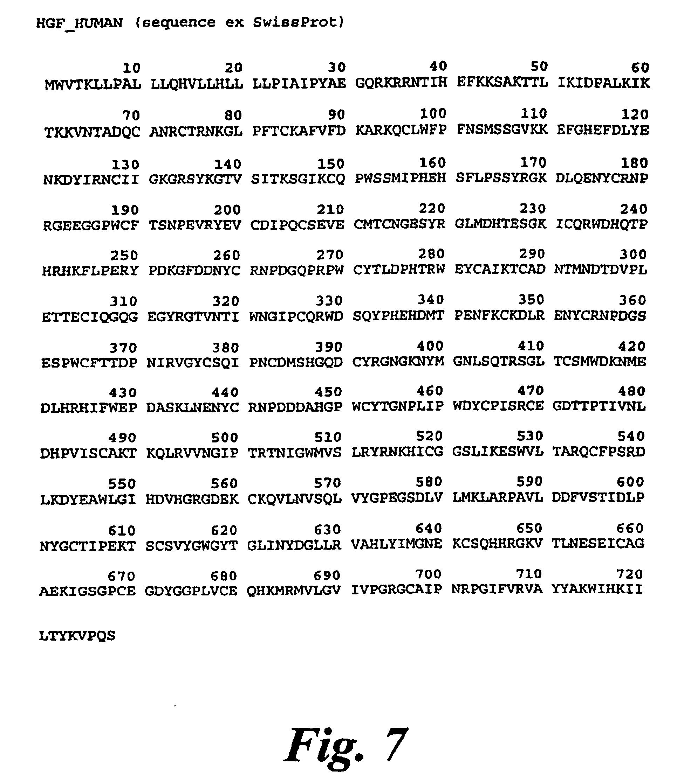

By "variant hepatocyte growth factor" we mean a hepatocyte growth factor (HGF) which varies in primary amino acid structure from a wild-type form. For example, the amino acid sequence of a wild-type form of human HGF is given in Figure 7. It is well known that certain polypeptides, especially those from humans, are polymorphic and it will be appreciated that some natural variation of the human HGF sequence may occur and that such human HGF molecules will be considered to be wild-type provided that the HGF binds heparan sulphate proteoglycan (HSPG) and is able to bind the HGF receptor and give rise to the known biological effect.

By "substantially incapable of binding HSPG" we include the meaning that the variant HGF binds HSPG significantly less well than wild-type HGF. It is preferred if the variant HGF binds HSPG at least 10-fold less well than wild-type HGF, preferably at least 20-fold less well, still more preferably 50-fold less well and most preferably at least 100-fold less well. Conveniently the HSPG-binding ability of wild-type HGF and variant HGF is measured with reference to the HSPGs produced by the cells in Example 1 described in Example 1.

Preferably, the variant HGF has a decreased affinity for heparin.

Typically, although not always, the variant HGF of the invention will have a decreased ability to bind both heparin and HSPG.

Conveniently, the variant HGF herein disclosed may be one that binds heparin significantly less well than wild-type HGF. It is preferred if the variant HGF binds heparin at least 10-fold less well than wild-type HGF, preferably at least 20-fold less well, still more preferably at least 50-fold less well and most preferably at least 100-fold less well. Conveniently, the heparin-binding ability of wild-type HGF and variant HGF is measured using the methods described in Example 1.

In any case, suitably, the variant HGF has substantially the same mitogenic and motogenic activities on target cells as wild-type HGF (at least those variants which are not antagonists of HGF).

Preferably the NK2 fragments of wild-type HGF or variant HGF are used to determine the binding affinity for heparin. Using the method of Example 1 wild-type human HGF has a molar dissociation constant (K_{d}) of ∼ 1 x 10⁻⁹M (1 nM) and the NK2 fragment of wild-type HGF has a molar dissociation constant (K_{d}) of around 2.3 nM with respect to heparin.

The naturally occurring form of NK2 is a product of alternative splicing of the primary HGF transcript and encodes the leader sequence (residues 1-31), the N domain containing the hairpin loop (residues 70-96), kringle 1 (residues 128-206), kringle 2 (residues 211-288) and an extra three residues after kringle 2 (residues 289-291).

By "capable of binding to the HGF receptor" we include the meaning that the variant HGF can bind with substantially the same affinity to a soluble form of the HGF receptor (MET) as wild-type HGF as described in Example 1. For the avoidance of doubt the variant HGFs of the invention include HGF variants that are capable of binding HGF receptor leading to either (a) activation of the receptor or (b) inhibition of receptor signalling by endogenous (or wild-type) HGF.

Preferably the variant HGF binds to the HGF receptor with an affinity of between 0.1- and 10-fold that of wild-type HGF.

Preferably, in some cases, the variants of HGF are not antagonists of HGF and for some variants the effect of binding the variant HGF to MET in a cell that contains MET and can respond to binding of HGF to MET is substantially the same as the effect of binding wild-type HGF to MET. Thus, in these cases the activity of the variant HGF which is not an antagonist of HGF and the activity of wild-type HGF on target cells *in vitro* is substantially the same.

Still preferably, as is described below, some variant HGF molecules are antagonists of HGF but these nevertheless fall within the scope of the invention.

As is discussed in more detail below, all of these types of molecules are believed to be useful in medicine and so are prepared in a form packaged and presented for use in medicine.

Before the present work it had not been shown that variants of HGF which are substantially incapable of binding heparin or HSPG but which are capable of binding to the HGF receptor have improved activities *in vivo* compared to wild-type or other variant forms of HGF. In particular at least some of the variants of the invention show unexpectedly slower clearance from serum than wild-type HGF and at least some of the variant HPG molecules of the invention unexpectedly induce DNA synthesis in adult liver to a higher degree than wild-type HGF.

By "HGF" we include single chain forms as well as forms which have been proteolysed to the two-chain form.

Preferably the variant HGF is a variant HGF wherein a positively-charged amino acid residue in the hairpin loop structure of wild-type HGF has been replaced with an amino acid residue with no charge or a negative charge.

The hairpin loop structure spans amino acid residues 70 to 96 of wild-type HGF.

By a "positively-charged amino acid residue" we mean Arg (R), Lys (K) or His (H). Histidine has a partial positive charge at physiological pH.

Arg and Lys are often called basic amino acid residues.

By a "negatively-charged amino acid residue" we mean Glu (E) or Asp (D). Apart from the given positively- or negatively-charged amino acid residues all other naturally occurring amino acid residues which are directly encoded by the genetic code have no charge.

Glu and Asp are often called acidic amino acid residues.

It is particularly preferred if the variant is one in which a positively-charged residue in the hairpin loop structure is replaced by a negatively-charged residue. It is therefore preferred if a Lys or Arg or His residue is replaced by a Glu or Asp residue. It is particularly preferred if a Lys or Arg residue is replaced by a Glu residue.

It will be appreciated that replacement of positively-charged residues by non-charged or negatively-charged residues is readily achieved by the well known methods of protein engineering as described below. However, variant HGF molecules which are substantially incapable of binding heparin or HSPG but which are capable of binding to the HGF receptor may be obtained by, for example, deleting positively charged amino acid residues present in the hairpin loop structure by protein engineering methods or, for example, by chemically modifying positively-charged residues in the hairpin loop structure. Protein engineering methods are preferred for replacement of amino acid residues; however, it may be possible to, for example, accomplish selective modification of arginine residues using cyclohexan-1,2-dione.

Preferably the HGF is a human HGF. The amino acid sequence of a wild-type human HGF is given in Figure 7. It is particularly preferred if the variant HGF is a variant human HGF in which positively-charged amino acid residues of the hairpin loop region are replaced by negatively-charged amino acid residues or amino acid residues with no charge. However, it will be appreciated that HGF from other mammalian species have an analogous hairpin loop structure which contains positively-charged residues and variants of these non-human HGF molecules in which the positively-charged amino acid residues are replaced by negatively-charged amino acid residues or by non-charged amino acid residues are included within the scope of the invention provided that such molecules are substantially incapable of binding heparin or HSPG but which are capable of binding to the HGF receptor.

In wild-type human HGF the positively-charged amino acid residues in the hairpin loop structure include R73, R76, K78, K85, K91, R93 and K94.

It is particularly preferred if the variant human HGF is one wherein at least Arg73 (R73) has been replaced by an amino acid residue with no charge or with a negative charge. Preferably the variant human HGF includes the mutation Arg73 Glu (R73E) or Arg73 Asp (R73D); most preferably Arg73 Glu (R73E).

It is also particularly preferred if the variant human HGF is one wherein at least Arg76 (R76) has been replaced by an amino acid residue with no charge or with a negative charge. Preferably the variant human HGF includes the mutation Arg76 Glu (R76E) or Arg76 Asp (R76D); most preferably Arg76 Glu (R76E).

An especially preferred embodiment of the invention is a variant human HGF wherein both amino acid residues Arg73 and Arg76 (R73 and R76) have been replaced independently of each other with an amino acid residue with no charge or with a negative charge. It is preferred that both Arg73 and Arg76 are replaced with a negatively-charged amino acid residue such as Glu (E) or Asp (D); it is particularly preferred if the variant human HGF comprises amino acid replacements Arg73 Glu and Arg76 Glu (R73E and R76E).

Conveniently, in addition to the replacement of Arg73 and Arg76 other positively charged amino acid residues in the hairpin loop structure are replaced with a negatively-charged amino acid residue or an amino acid residue with no charge. Suitably, in addition to the replacement of Arg73 and Arg76 of human HGF, Arg93 (R93) and/or Lys78 (K78) are replaced with a negatively-charged amino acid residue or an uncharged amino acid residue. Preferably Arg93 (R93) and/or Lys78 (K78) are replaced with Glu (E) or Asp (D), most preferably Glu (E).

Thus, a preferred embodiment of the invention is a variant human HGF comprising amino acid replacements R73E, R76E and R93E, and a variant human HGF comprising amino acid replacements R73E, R76E and K78E.

The variant HGF molecules of the invention include HGF molecules which, in addition to containing the mutations referred to above, may also be modified further in order to modulate the biological activity of the molecule (for example, in order to make an HGF antagonist) or they may be modified further in order to facilitate synthesis and/or purification of the variant HGF molecule. However, these further modifications retain the given properties of the variant HGF molecule namely that the variant is substantially incapable of binding HSPG or heparin as the case may be but is capable of binding to the HGF receptor.

Suitably the variant HGF may comprise a "tag" peptide sequence which may facilitate its purification. Typically, the tag comprises a binding site for a component which may be immobilised in order to allow a solid phase purification of the variant HGF following binding of the variant HGF to the component. The tag may be, for example, the Hisₙ sequence (n > 4) which binds to Ni²⁺ ions, or it may be an epitope for a monoclonal antibody such as the well known Myc-tag epitope. Other possibilities are well known in the art.

It is preferred, however, if the variant human HGF consists of a wild-type HGF with amino acid replacements Arg73 Glu and Arg76 Glu (R73E, R76E) or a wild-type HGF with amino acid replacements Arg73 Glu, Arg76 Glu and Arg93 Glu (R73E, R76E, R93E) or a wild-type HGF with amino acid replacements Arg73 Glu, Arg76 Glu and Lys78 Glu (R73E, R76E, K78E).

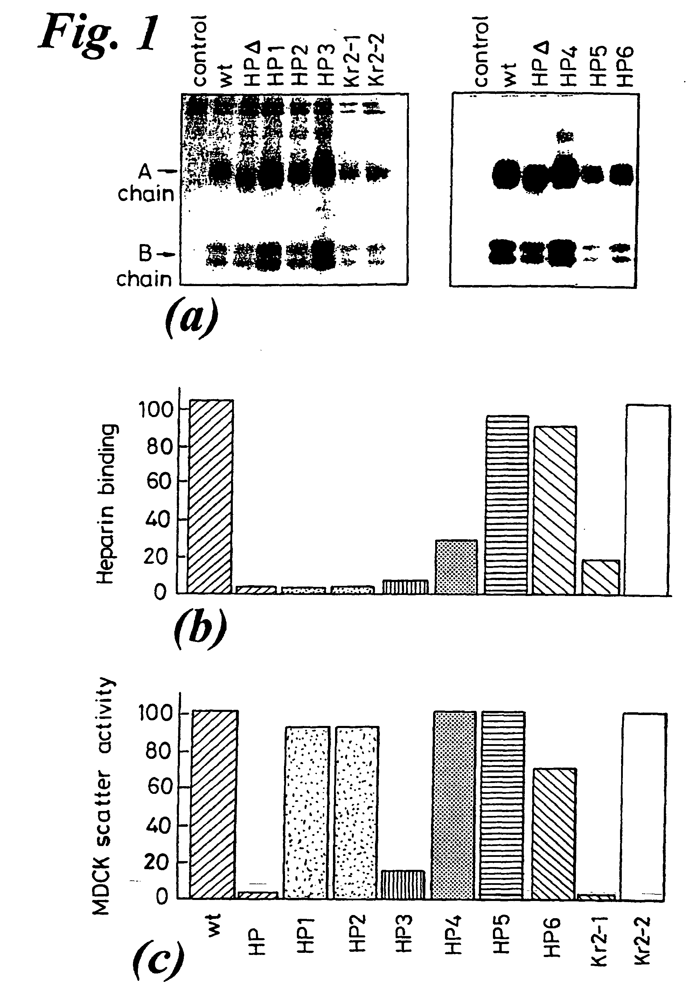

The variant human HGF with amino acid replacements R73E, R76E and R93E is called HP1 in Example 1 and the variant human HGF with amino acid replacements R73E, R76E and K78E is called HP2 in Example 1 (see legend to Figure 1).

It is also preferred if the variant human HGF comprises mutations of L80 and/or F82. In particular a preferred variant human HGF comprises the mutations L80S and F82Q. The variant human HGF with amino acid replacements L80S and F82Q is called HP4 in Example 1 and is a variant HGF for use in medicine of the invention. It will be appreciated that it may be beneficial to introduce one or more of these mutations into a variant HGF which also contains one or more of the mutations in the hairpin loop which remove the positive charge as herein described.

In addition to the aforementioned mutations it may be advantageous to replace other positively-charged amino acid residues in the human HGF molecule with non-charged or negatively-charged amino acid residues. For example, it may be advantageous to introduce one or more replacements of K91 and K94 (in the hairpin structure) and H241, R242, K244 and R249 (in kringle 2 domain).

A variant human HGF which consists of wild-type HGF in which Lys85 is replaced by Glu (K85E; HP5 - see legend to Figure 1) is not a variant of the invention since it behaves essentially like wild-type HGF. However, this mutation may be beneficially included with the other mutations herein disclosed.

The variant HGF is preferably one which, in relation to its MET binding ability and its ability to transduce signals into the cell, has substantially the same activity as wild-type HGF.

However, it is also preferable that the variant HGF is one which antagonises the action of wild-type HGF. Variant HGF molecules which antagonise the action of wild-type HGF are known in the art. For example, WO 92/05184 and WO 96/40914 relate to truncated forms of HGF which specifically antagonise or partially antagonise the activity of wild-type HGF. WO 93/23541, US 5,547,856, US 5,316,921 and US 5,580,963 relate to variant HGF molecules which are resistant to proteolytic cleavage by enzymes capable of in *vivo* conversion of HGF into its two-chain form. All of these patent applications and patents are incorporated by reference.

Thus, it is preferred if the variants described in these patent applications and patents are further modified in the way described herein by, for example, making additional mutations which make the variant HGF substantially incapable of binding heparin or HSPG but which is capable of binding to the HGF receptor.

Preferably the mutations disclosed herein are combined with the mutations which confer resistance to proteolytic cleavage by enzymes capable of in *vivo* conversion of HGF into its two-chain form such as those described in US 5,316,921. More preferably, these mutations, which may be combined with the mutations disclosed herein in relation to the hairpin loop structure, are ones in which an amino acid alteration at or adjacent to any of amino acid positions 493, 494, 495 and 496 of wild-type human HGF sequence.

Thus, preferred variant HGF molecules of the invention which are believed to be antagonists of wild-type HGF are, for example, those comprising the amino acid replacements R73E, R76E and R93E, or R73E, R76E and K78E and any one or more of replacements of amino acid positions 493, 494, 495 and 496 of wild-type human HGF.

As discussed above the aforementioned variant HGF molecules are useful in medicine.

A further aspect of the invention provides a pharmaceutical composition comprising a variant HGF as defined in the first aspect and a pharmaceutically acceptable carrier.

The aforementioned compounds of the invention or a formulation thereof may be administered by any conventional method including oral and parenteral (eg subcutaneous or intramuscular) injection. The treatment may consist of a single dose or a plurality of doses over a period of time.

Whilst it is possible for a compound of the invention to be administered alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen free.

The formulations may conveniently be presented in unit dosage form and may be prepared by any of the methods well known in the art of pharmacy. Such methods include the step of bringing into association the active ingredient (compound of the invention) with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg povidone, gelatin, hydroxypropylmethyl cellulose), lubricant, inert diluent, preservative, disintegrant (eg sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

Localised delivery may be desirable where possible; for example when delivery is desired to the liver it may be via the hepatic artery.

A further aspect of the invention provides a method of treating a patient in need of or who would benefit from treatment with a hepatocyte growth factor or an antagonist thereof the method comprising administering to the patient an effective amount of a variant HGF as defined in the first aspect of the invention.

It will be appreciated that with the advances in gene therapy it may be possible to administer the variant HGF via administration of a genetic construct which encodes the variant HGF. This is specifically included in the method of treatment of the invention.

The therapeutic use of wild-type and particular variant HGF molecules distinct from those disclosed herein has been described previously. For example, studies in mice suggest that HGF may function as a tumour suppressor in the early stages of liver carcinogenesis (Santoni *et al* (1996) *Proc. Natl. Acad. Sci. USA* **93**, 9577-9582), and HGF is reported to inhibit the growth of Hep G2, HCC, B6/F1 melanoma and KB squamous carcinoma cells *in vitro* (Tajima *et al* (1991) *FEBS Lett.* **291**, 229-232). HGF prevents onset and progression of hepatic fibrosis/cirrhosis in rats and completely abrogates death caused by severe hepatic cirrhosis and dysfunction (EP 0 456 188 and Matsuda *et al* (1995) *J. Biochem.* **118**, 643-649). HGF enhances the efficiency of retroviral transduction of primary hepatocytes (Pages *et al* (1996) *Biochem. Biophys. Res. Comm.* **222**, 726-731).

HGF stimulates liver regeneration in 70%-hepatectomised rats (Ishii *et al* (1995) *J. Biochem.* **117**, 1105-1112). HGF has been proposed as a therapy against vascular smooth muscle cell proliferation (Nakamura *et al* (1995) *Biochem. Biophys. Res. Comm.* **215**, 483-488, and decrease in local HGF concentrations due to high D-glucose may be a trigger of endothelial injury in diabetes, potentially resulting in the progression of atherosclerosis (Nakamura *et al* (1997) *Diabetes* **46**, 138-142).

HGF is a potent angiogenesis factor *in vivo* (Rosen *et al* (1993) *Symp.* *Soc. Exp. Biol.* **47**, 227-234) and thus may have utility in wound healing.

HGF is reported to enhance repair of intestinal epithelial erosions/ulcerations (Nusrat *et al* (1994) *J. Clin. Invest.* **93**, 2056-2065). HGF has been proposed to block side effects of radio/chemotherapy (WO 93/08821; WO 95/25537), as a remedy for cranial nerve disorders (WO 95/07709), as a remedy for cartilage disease (WO 96/05855), to relieve side effects caused by immunosuppressants (WO 95/25537) and as a therapeutic agent for renal disease (EP 0 462 549). See also Matsumoto & Nakamura, Hepatocyte Growth Factor, pages 450-474 In: Acute Renal Failure, New Concepts and therapeutic strategies, ed. M.S. Goligorsky & J.H. Stein, Churchill Livingstone, New York. All of these documents are incorporated herein by reference.

HGF, or variants thereof, is reported to be useful in the treatment of various other disorders. WO 97/09997 suggests that HGF may be useful in treating cystic fibrosis. WO 97/12628 suggests that HGF may enhance resurfacing of blood vessels damaged or traumatized, for instance, by vascular surgery or angioplasty, and WO 97/12629 suggests that HGF may be useful in enhancing angiogenesis. The α fragment of HGF may act as an antagonist which specifically inhibits the ability of cancer cells to invade or metastasise by means of HGF (see WO 97/16205), whereas in WO 96/32960 HGF is employed as the active ingredient which exerts the effect of suppressing cytotoxicity in an ischaemic model and, therefore, is efficacious as a preventive and/or remedy for ischaemic diseases.

WO 95/29694 indicates that HGF can accelerate collagen hydrolysis to treat fibrosis and other diseases caused by a lowered collagenase activity, and EP 0 661 995 suggests that HGF may be used in the prevention of the establishment of, or progress of, liver damage in patients at risk of liver damage. HGF variants which prevent HGF from binding to MET are proposed for use in a method of preventing tumour cell metastasis (see, for example, EP 0 805 203).

In addition, WO 98/00543 describes various HGF receptor agonists, and WO 97/07824 indicates that the HGF gene may be employed, with the use of liposomes, in gene therapy.

The variant HGF molecules which bind MET and which transduce cellular signals in substantially the same way as wild-type HGF are believed to be therapeutically useful in the same way as wild-type HGF but since the variant HGF molecules are substantially incapable of binding HSPG or heparin the variant molecules are believed to have a superior effect *in vivo* since, for example, the variant HGF molecules are believed to give greater tissue penetration, hence the ability to reach cell or tissue compartments which exogenous, wild-type HGF cannot reach effectively (or would require a much higher dose. Accordingly, it is believed that the therapeutic efficacy of at least some of the variant HGF molecules of the invention is higher than for wild-type HGF and that a lower therapeutic dose can be used which may result in lower side effects. Thus, this class of variant HGF molecules are believed to be useful as a cancer therapeutic, in treating human liver fibrosis/cirrhosis, as a therapeutic agent in hepatectomy and liver injury, as an adjuvant for gene therapy of the liver, in therapy against vascular smooth cell muscle cell proliferation, to enhance repair of intestinal epithelial erosions/ulcerations, as an angiogenesis factor, to block side effects of radio- or chemotherapy, as a remedy for cranial nerve disorder, as a remedy for cartilage disease, to relieve side effects caused by immunosuppressants and as a therapeutic agent for renal diseases. These variant HGF molecules may also be useful in treating chronic skin wounds.

The variant HGF molecules of the invention are believed to be useful in treating cancer in order to prevent or retard spreading and metastasis.

A further aspect of the invention provides the use of a variant HGF as defined in the first aspect of the invention in the manufacture of a medicament for treating a patient in need of or who would benefit from treatment with a HGF or an antagonist.

A further aspect of the invention provides a variant hepatocyte growth factor (HGF) which is substantially incapable of binding heparin or HSPG but which is capable of binding to the HGF receptor provided that the variant HGF is not a variant of human HGF in which the replacements (a) R73E, R76E and R93E or (b) R73E and R76E or (c) K91E, R93E and K94E have been made.

The preferences for the variant HGF molecules of this aspect of the invention are the same as those for the first aspect of the invention.

A further aspect of the invention provides a polynucleotide encoding a variant hepatocyte growth factor (HGF) which is substantially incapable of binding heparin or HSPG but which is capable of binding to the HGF receptor provided that the variant HGF is not a variant of human HGF in which the replacements (a) R73E, R76E and R93E or (b) R73E and R76E or (c) K91E, R93E and K94E have been made.

The polynucleotide of this aspect of the invention can be readily made, for example by site-directed mutagenesis using mismatched oligonucleotides or by using the polymerase chain reaction or by *de novo* polynucleotide synthesis or by any other methods. Such methods are well known in the art of molecular biology and at least some of the methods are described in detail in Sambrook *et al* (1989), Molecular Cloning, a laboratory manual, Cold Spring Harbor Laboratory, Cold Spring Harbor, New York.

A further aspect of the invention provides a vector comprising a polynucleotide of the invention and a still further aspect of the invention provides a host cell comprising a polynucleotide or vector of the invention.

The polynucleotide or vector may be DNA or RNA; preferably it is DNA.

A variety of methods have been developed to operably link DNA to vectors via complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion as described earlier, is treated with bacteriophage T4 DNA polymerase or E. *coli* DNA polymerase I, enzymes that remove protruding, 3'-single-stranded termini with their 3'-5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA encoding the polypeptide of the invention is to use the polymerase chain reaction as disclosed by Saiki *et al* (1988) *Science* **239**, 487-491.

In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

The DNA (or RNA) is then expressed in a suitable host to produce a variant HGF (polypeptide constituting the compound of the invention) of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter et *al,* 4,530,901 issued 23 July 1985 to Weissman, 4,582,800 issued 15 April 1986 to Crowl, 4,677,063 issued 30 June 1987 to Mark *et al,* 4,678,751 issued 7 July 1987 to Goeddel, 4,704,362 issued 3 November 1987 to Itakura *et al,* 4,710,463 issued 1 December 1987 to Murray, 4,757,006 issued 12 July 1988 to Toole, Jr. *et al,* 4,766,075 issued 23 August 1988 to Goeddel *et al* and 4,810,648 issued 7 March 1989 to Stalker, all of which are incorporated herein by reference.

The DNA encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example E. *coli* and *Bacillus subtilis*)*,* yeasts (for example *Saccharomyces cerevisiae*)*,* filamentous fungi (for example *Aspergillus*), plant cells, animal cells and insect cells.

The vectors include a prokaryotic replicon, such as the ColE1 *ori,* for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as *E*. *coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are pUC18, pUC19, pBR322 and pBR329 available from Biorad Laboratories, (Richmond, CA, USA) and p*Trc99*A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3*. Plasmids pRS413-416 are Yeast Centromere plasmids (YCps).

The present invention also relates to a host cell transformed with a polynucleotide vector construct of the present invention. The host cell can be either prokaryotic or eukaryotic. Bacterial cells are preferred prokaryotic host cells and typically are a strain of *E. coli* such as, for example, the *E. coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast and mammalian cells, preferably vertebrate cells such as those from a mouse, rat, monkey or human fibroblastic cell line. Yeast host cells include YPH499, YPH500 and YPH501 which are generally available from Stratagene Cloning Systems, La Jolla, CA 92037, USA. Preferred mammalian host cells include Chinese hamster ovary (CHO) cells available from the ATCC as CCL61, NIH Swiss mouse embryo cells NIH/3T3 available from the ATCC as CRL 1658, and monkey kidney derived COS-1 cells available from the ATCC as CRL 1650.

Transformation of appropriate cell hosts with a DNA construct of the present invention is accomplished by well known methods that typically depend on the type of vector used. With regard to transformation of prokaryotic host cells, see, for example, Cohen *et al* (1972) *Proc. Natl. Acad. Sci. USA* **69**, 2110 and Sambrook *et al* (1989) *Molecular Cloning, A Laboratory Manual,* Cold Spring Harbor Laboratory, Cold Spring Harbor, NY. Transformation of yeast cells is described in Sherman *et al* (1986) *Methods In Yeast Genetics, A Laboratory Manual,* Cold Spring Harbor, NY. The method of Beggs (1978) *Nature* **275**, 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming cells and is well known in the art for transforming yeast cell, bacterial cells and vertebrate cells.

For example, many bacterial species may be transformed by the methods described in Luchansky *et al* (1988) *Mol. Microbiol.* **2**, 637-646 incorporated herein by reference. The greatest number of transformants is consistently recovered following electroporation of the DNA-cell mixture suspended in 2.5X PEB using 6250V per cm at 25µFD.

Methods for transformation of yeast by electroporation are disclosed in Becker & Guarente (1990) *Methods Enzymol.* **194**, 182.

Successfully transformed cells, ie cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern (1975) *J. Mol. Biol.* **98**, 503 or Berent *et al* (1985) *Biotech.* **3**, 208. Alternatively, the presence of the protein in the supernatant can be detected using antibodies as described below.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium.

It is preferred if the host cells are insect cells and the expression is a baculovirus expression.

It is also preferred if the host cells are *Pichia pastoris.*

It will be appreciated that, since it is useful to use the variant HGF molecules, or at least polynucleotides encoding them, in gene therapy, the invention also includes methods of introducing the said polynucleotides into the cells of the patient.

The invention will now be described in detail with reference to the following Examples and Figures in which
Fig. 1 shows the heparin binding and MDCK scatter activity of wt- and mutant forms of HGF/SF.
   The following mutants were generated either in the hairpin structure (HP) or kringle 2 (Kr2) of HGF/SF: HP1: R73E, R76E, R93E; HP2: R73E, R76E, K78E; HP3: K91D, R93E, K94D; HP4: L80S, F82Q; HP5: K85E; HP6: D90K; Kr2-1: H241S, R242E, K244E, R249G and Kr2-2: R281G, W282G, Y284A).
   a) *Expression of wt- and mutant forms of HGF*/*SF.* The figure shows an SDS gel under reducing conditions of proteins present in the supernatant of Neuro 2a cells transiently transfected with HGF/SF cDNAs and labelled with ³⁵S-cysteine. The migration of the 60 kDa A chain and the 32/34 kDa B chain is indicated. Control lanes are supernatants of Neuro 2a cells transfected with vector alone. The mutant HPΔ has the complete hairpin structure deleted (residues 68-100) and therefore the mobility of the A chain is slightly increased compared to wt-HGF/SF.
   b) *Binding of 20 ng*/*ml of wt- or mutant HGF*/*SF proteins to wells coated with 200 µg*/*ml of heparin.* The data are expressed as percentage of wt-HGF/SF. Deletion of the hairpin structure (HPΔ) reduced heparin binding to < 5%. Similarly, all mutants in which basic amino acid residues were exchanged (HP1, HP2, HP3) showed strongly reduced heparin binding. Mutations outside the positively charged surface (HP4, HP5, HP6) either had a less pronounced effect (HP4) or no effect (HP5 and HP6). Substitution of positively charged amino acids on the surface of kringle 2 (Kr2-1) reduced heparin-binding to 18% of wt-HGF/SF, whereas deletion of the lysine-binding pocket (Kr2-2) had no effect.
   c) *Scatter of MDCK colonies by wt- and mutant HGF*/*SFs expressed* *in Neuro 2a cells.* The data are expressed as percentage of wt-HGF/SF. Deletion of the hairpin structure (HPΔ) abolished biological activity but point mutations of positively charged amino acids in the hairpin structure had variable effects on biological activity. Thus the HP1 and HP2 mutants had biological activities comparable to wt-HGF/SF whereas the activity of mutant HP3 was only 17% of wt-HGF/SF. The kringle 2 mutants showed either a severe loss of biological activity (Kr2-1) or no change (Kr2-2).

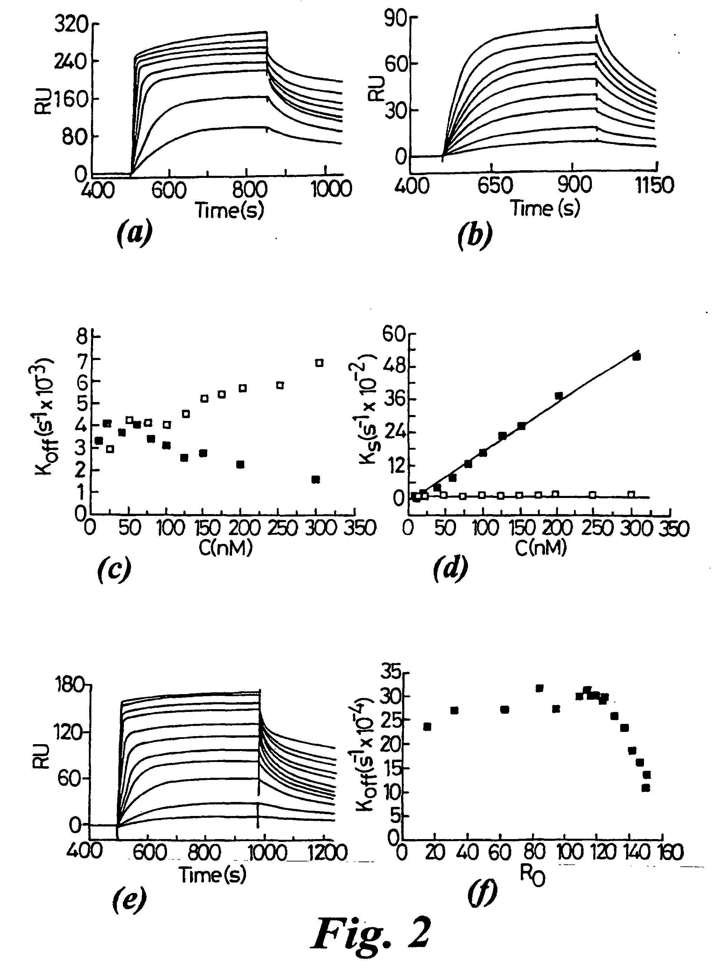

Fig. 2. shows a BIAcore analysis of wt-NK2 and HP1-NK2 binding to immobilised heparin
   Panel a shows a sensorgram overlay of wt-NK2 (10-300 nM) binding to a heparin surface (maximum binding capacity 340 RU). Panel b shows a corresponding set of sensorgrams for HP1-NK2 (10-300 nM) (surface capacity ca 170 RU). A lower surface capacity and longer injection time were used for HP1-NK2 to allow for the great differences in association rates. The spikes at the beginning and the end of the sample pulses are artefacts resulting from subtraction of the curves from the blank surface (not shown) and are due to small, irresolvable timing errors. Panel c shows a k_{off} vs concentration (C, nM) and panel d shows a plot of the observed rate constant kₛ *vs* concentration (C, nM) for wt-NK2 (solid squares) and HP1-NK2 (open squares).

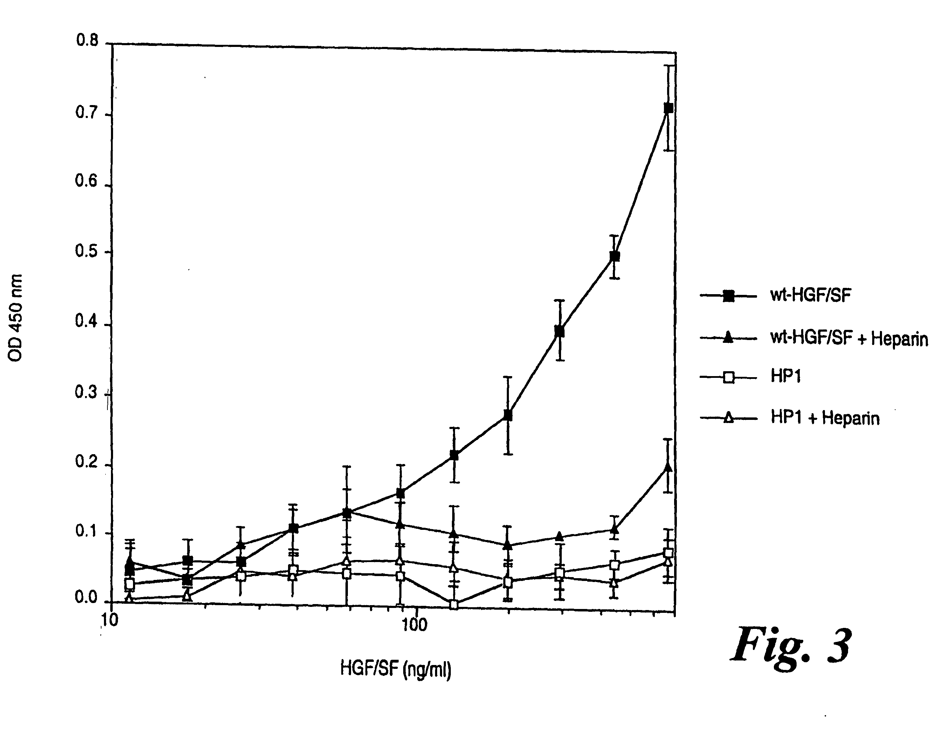

Fig. 3 shows the total binding of wt-HGF/SF and HP1 to mink lung cells.
   The total binding of wt-HGF/SF and HP1 to mink lung cells in a 96 well plate is shown. wt-HGF/SF showed strong concentration-dependent binding to the cell surface of mink lung cells (solid squares) which could be competed by addition of soluble heparin at 0.1 mg/ml (solid triangles). In contrast, the binding of HP1 was negligible at concentrations up to 675 ng/ml (open squares) and was not affected by the addition of heparin (open triangles).

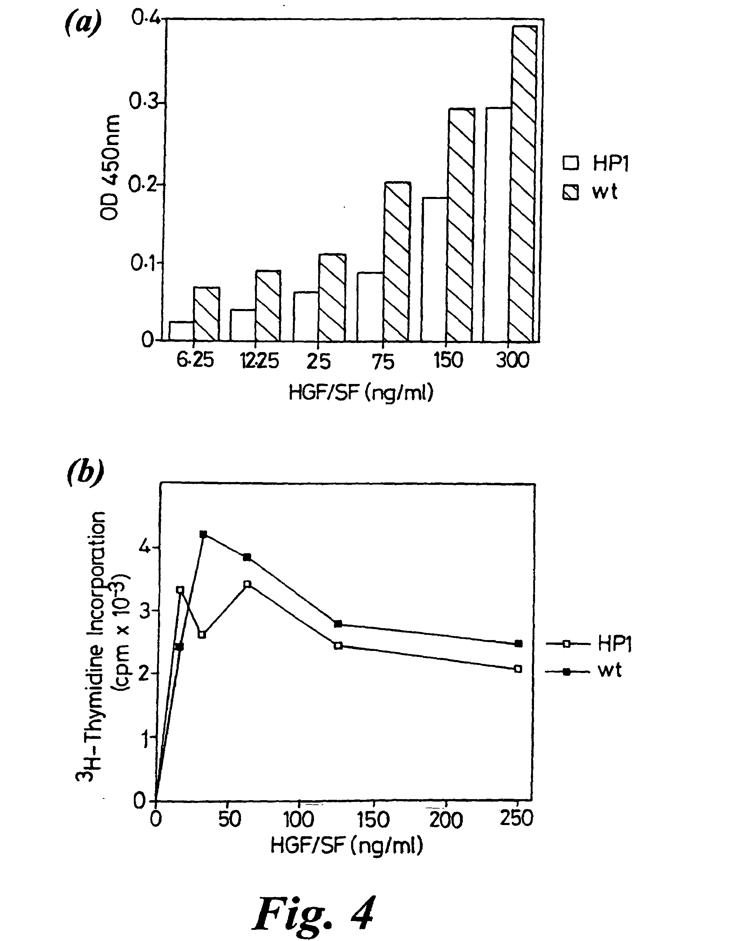

Fig. 4 shows the binding of wt-HGF/SF and HP1 to a soluble form of the MET-receptor (MET-Fc) and effect of wt-HGF/SF and HP1 on DNA synthesis in mink lung cells.
   a) *Binding of wt-HGF*/*SF (solid bars) and HP1 (open bars) to a soluble form of the MET receptor (MET-Fc)*. Immobilised wt-HGF/SF or HP1 were incubated with 200 ng/ml of MET-Fc and bound MET-Fc was detected with an anti-human IgG antibody. The binding of the HP1 mutant was only slightly lower than for wt-HGF/SF.
   b) *Effect of wt-HGF*/*SF (solid squares) and HP1 (open squares) on DNA synthesis in mink lung cells.* 10,000 serum-starved mink lung cells were incubated with the indicated concentrations of wt HGF/SF or HP1. After 24 hours, ³H-thymidine was added and the amount of radioactivity incorporated into DNA was measured in duplicate samples. The figure shows DNA synthesis induced by wt-HGF/SF or HP1 after subtraction of background counts (³H-thymidine incorporated by cultures incubated in control medium). The stimulation induced by HP1 was only slightly lower than for wt-HGF/SF.

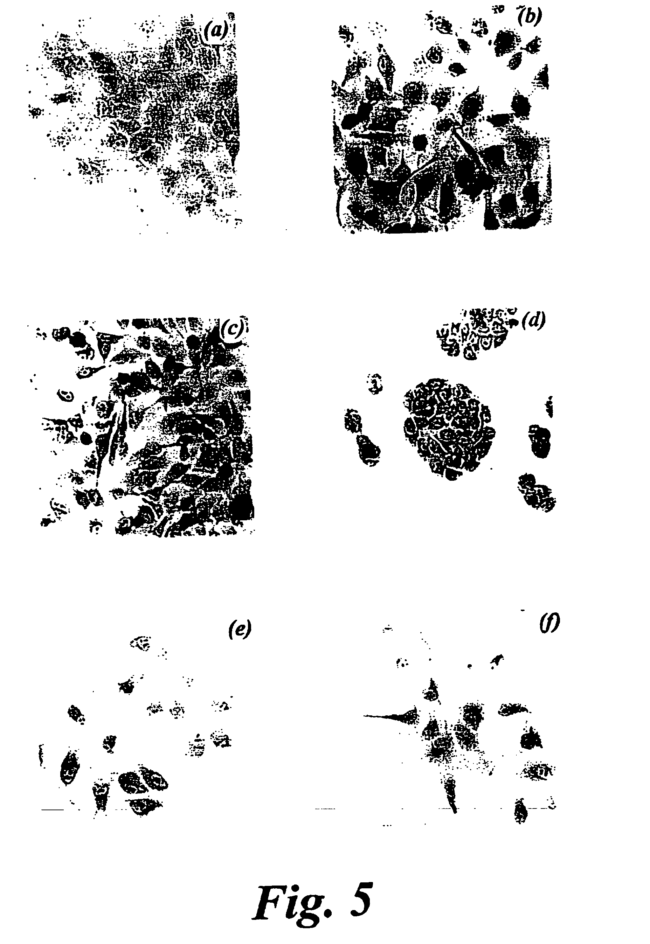

Fig. 5 shows the effect of wt-HGF/SF and HP1 on the morphology of mink lung and MDCK cells.
   The figure compares mink lung cells (panels a, b, and c) and MDCK cells (panels d, e and f) cultured in the absence of factors (a and d) or after overnight incubation with 20 ng/ml of wt-HGF/SF (b and e) or HP1 (c and f). In control cultures (a and d) cells exhibited a typical epithelial morphology. The effects of wt-HGF/SF and HP1 on cell dissociation and morphology were undistinguishable.

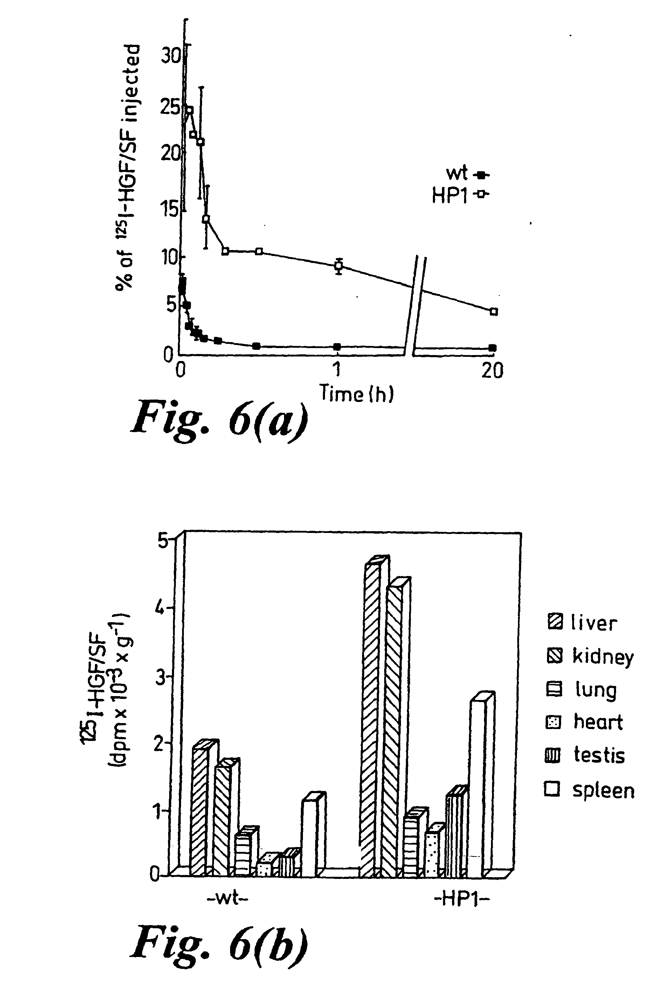

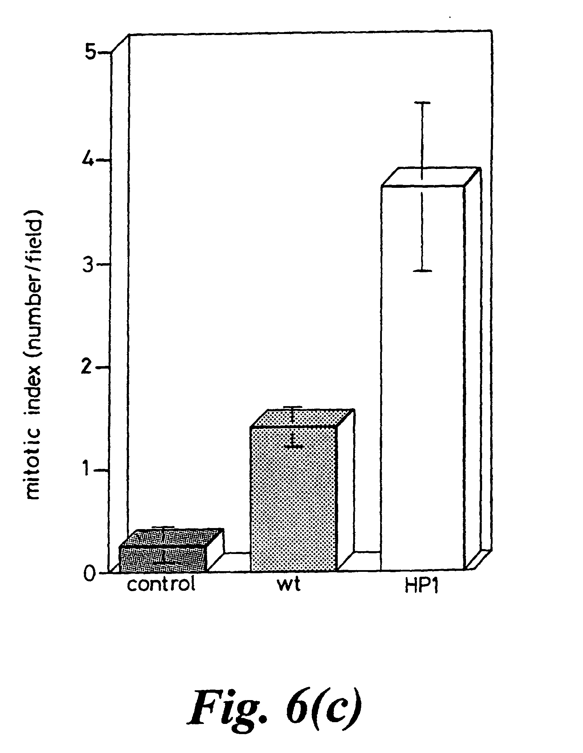

Fig. 6 shows the clearance, tissue distribution and activity in *vivo* of wt-HGF/SF and HP1.
   a) *Clearance curves of* ^{*125*}*I -labelled wt-HGF*/*SF and HP1 from rat serum.* Six adult rats were injected either with ¹²⁵I -labeled wt-HGF/SF or HP1 in the jugular vein and blood samples taken at the times shown. The concentration of wt-HGF/SF (solid squares) and HP1 (open squares) remaining in serum (TCA insoluble radioactivity) is shown as a fraction of the total radioactivity injected. For each time point the level of mutant HP1 in blood was at least 5 times higher than for wt-HGF/SF.
   b) *Distribution of* ^{*125*}*I-labelled wt-HGF*/*SF and HP1 in rat tissues.* Two hours after injection, two animals were sacrificed and, after perfusion, several organs (liver, kidney, lung, heart, testis spleen) were homogenised and the amount of TCA-insoluble radioactivity measured. The level of tissue radioactivity was 2-3 fold higher for HP1 than for wt-HGF/SF, although the pattern of organ distribution was the same with the two proteins.
   c) *Stimulation of DNA synthesis in mouse liver by wt-HGFlSF and HP1.* wt-HGF/SF and HP1 were administered intravenously over a period of 3 days (total dose 277.5 *µ*g/animal distributed in 5 injections). At the time in which the animals received the last dose of factor, BrdU was injected intraperitoneally (75 *µ*g/g body weight) and 12 hours later the animals were sacrificed. BrdU-labelled cells were counted on frozen liver sections (at least 8 different sections and a total of 120 fields per animal).
      Data are the mean ± sd for 4 (wt- HGF/SF) or 3 (HP1) animals.
Figure 7 shows the amino acid sequence of human hepatocyte growth factor from the Swiss Prot database accession no. P14210. Nucleotide sequences encoding HGF or information relating to such sequences are given in Seki *et al* (1991) *Gene* **102**, 213-219; Miyazawaka *et al* (1989) *Biochem. Biophys. Res. Comm.* **163**, 967-973; Seki *et al* (1990) *Biochem. Biophys. Res. Comm.* **172**, 321-327; Nakamura *et al* (1989) *Nature* **342**, 440-443; Weidner *et al* (1991) *Proc. Natl. Acad. Sci. USA* **88**, 7001-7005; Yushiyama *et al* (1991) *Biochem. Biophys. Res. Comm.* **175**, 660-667; and Lokker *et al* (1992) *EMBO J.* **11**, 2503-2510 all of which are incorporated herein by reference.
   *Abbreviations:* EGF: epidermal growth factor; FGF: fibroblast growth factor; HB-EGF: heparin-binding EGF; HGF/SF: hepatocyte growth factor/scatter factor; HSPGs: heparan sulphate proteoglycans; MES: 2-[N-morpholino]ethane sulfonic acid; NK2: a truncated variant of HGF/SF corresponding to the N terminal domain, kringle 1 and kringle 2; SPR; surface plasmon resonance; VEGF: vascular endothelial growth factor.

### Example 1: Engineered mutants of HGF/SF with reduced binding to heparan sulphate proteoglycans and improved activity in vivo

### Summary

HGF/SF is a high molecular weight, heparin-binding, polypeptide growth factor that plays important roles in mammalian development and tissue regeneration. In order to identify the amino acids involved in receptor or heparin binding, we have engineered a number of HGF/SF mutants in which several clusters of solvent-accessible residues in the hairpin structure of the N-terminal domain or in kringle 2 have been replaced. Two of the mutants (HP1: R73E, R76E, R93E and HP2: R73E, R76E, K78E) were of particular interest as they exhibited greatly decreased affinity for heparin but full mitogenic and motogenic activities on target cells in culture. The affinity of HP1 for heparin was more than 50 fold lower compared to wild type HGF/SF, as established from the binding kinetics of the NK2 fragments of the two proteins. The change in affinity in HP1 was the result of a decreased on-rate (∼ 5 x 10⁴ M⁻¹ s⁻¹*vs* 1.75 x 10⁶ M⁻¹ s⁻¹) and was paralleled by decreased binding to cell-surface and matrix heparan sulphate proteoglycans. Important differences were observed in the behaviour of wild type HGF/SF and HP1 *in vivo:* the mutant protein exhibited a delayed clearance from blood, higher tissue levels and a 2.7 fold higher activity in inducing DNA synthesis in normal, adult murine liver. Thus we have engineered mutant forms of HGF/SF which, by virtue of decreased affinity for heparan sulphate, exhibit greater activity *in vivo.* These results have implications for understanding the role of heparan sulphate proteoglycans in HGF/SF activity and for therapeutic applications of the growth factor.

In order to dissect the receptor and heparin binding sites in HGF/SF, we generated three-dimensional models of the individual domains (29) to help design a number of site-specific HGF/SF mutants. The available X-ray structures of antithrombin (30) and FGF-heparin complexes (31) indicate that the heparin binding sites are composed primarily of clusters of positively charged residues making electrostatic contact with negatively charged groups in the glycosaminoglycan. We therefore searched the surface of the hairpin structure and kringle 2 of HGF/SF for clusters of positively charged amino acids, identified three such clusters (two in the hairpin structure and one in kringle 2) and introduced specific mutations at these sites.

### Materials and Methods

### Cell lines

Cell lines were either purchased from ATCC or previously described in (7) and (32). Cells were routinely grown in DMEM supplemented with 10% fetal calf serum at 37°C and 10% CO₂.

### In vitro mutagenesis of the hairpin structure and kringle 2

A double stranded oligonucleotide (5'-CA CAG TCA GGA CAT CAT CAT CAT CAT CAT TAA GGA TCC TCT AGA GGT AC -3') coding for six histidine residues and a stop codon was cloned into the Kpn I restriction site of the 2.2 kb Bam HI/Kpn I fragment of the HGF/SF cDNA. In initial experiments, a cDNA encoding wt- HGF/SF fused to a C-terminal heamagglutinin tag was used (26). For the generation of point mutants of the hairpin structure or kringle 2, codon substitutions were introduced into the cDNA by annealing mismatched primers in PCR reactions. The PCR fragments were cloned into the cDNA and mutations confirmed by sequencing. The point mutants generated in this way are listed in the legend to Fig. 1. For deletion of the hairpin structure, an additional Pst I restriction site was created at nt 300 and, by partial digestion, a fragment coding for amino acids 68 to 100 of HGF/SF was deleted.

### Transient expression of wt and mutant HGF/SF in Neuro 2a cells

cDNAs encoding mutant HGF/SFs (10 µg each) were transiently transfected into Neuro 2a cells as described (26). For immunoprecipitation, one day after transfection the Neuro 2a cells were labelled overnight with 20 µCi/ml of ³⁵S-cysteine (Amersham) and HGF/SF was immunoprecipitated from the supernatant (1.5 ml) using 2 µg of anti-HGF/SF antibody (clone 34) and 100 ml of protein A-Sepharose 4B beads. The precipitate was washed three times with 50 mM Tris-Cl pH 8.5, 100 mM NaCl, 1 mM EDTA, 1 mg/ml ovalbumin and 5 g/l Triton X-100 and used for SDS gel electrophoresis and phosphoimager analysis.

### Expression of full length wt-HGF/SF and HP1 in insect cells

For large scale production of wt -HGF/SF and HP1 the corresponding cDNAs were fused to a C-terminal histidine tag and cloned into the Baculovirus expression vector pVL1393. Recombinant virus was generated by cotransfection of 2 µg of expression plasmid and 0.5 µg Baculogold-DNA (Pharmingen, La Jolla, USA), plaque purified, propagated and used to infect 5 litre batches of SF9 cells. wt-HGF/SF and HP1 were purified from the supernatant after extensive dialysis against PBS and affinity chromatography on 5 ml Ni²⁺-chelate columns (Qiagen, Germany) using a linear gradient of imidazole (0-0.4 M in 0.1 M NaCl, 50 mM MES pH 6.0) followed by cation exchange chromatography (8). The purity of the preparations was typically 80-90% from Coomassie stained SDS-gels.

### Expression of wt-NK2 and HP1-NK2 in P. pastoris

cDNAs encoding wt-NK2 or HP1-NK2 were generated by PCR amplification of a human HGF/SF cDNA and cloned into the *P. pastoris* expression vector pPIC 9K (Invitrogen Inc.) creating an in frame fusion with the α-mating signal peptide of *S*. *cerevisiae.* Linearised, recombinant pPIC 9K plasmids encoding wt-NK2 and HP1-NK2 plasmids were used for transformation of the GS115, *his* strain of *P. pastoris* by spheroplasting. Transformed clones were initially selected on his plates and subsequently on G418 plates for multicopy integration (33). Selected clones were used for large-scale expression of the recombinant proteins in shake flasks. The culture supernatant was harvested 72 hours after induction and the supernatant was clarified by centrifugation followed by filtration. wt-NK2 was initially purified on heparin-Sepharose4B (Pharmacia) followed by ion-exchange chromatography on S-Sepharose (Pharmacia) using a gradient of NaCl (0.25-1.00 M) in 0.05 M MES, pH 6.0. HP1-NK2 was purified directly by ion-exchange chromatography on S-Sepharose. Monomer and dimer species of wt-NK2 and HP1 NK2 were separated by size-exclusion chromatography on a Superdex75 column (Pharmacia) in 0.05 MES, 0.25 M NaCl, buffer, pH 6.0. The yield of biologically active protein was between 5-10 mg/litre.

### Heparin and MET ELISAs

For heparin ELISA, 96 well plates (Nunc, Multisorp) were incubated with 200 µl/well of high molecular weight heparin (200 µg/ml, Sigma H3393). After blocking, purified HGF/SF and supernatants of Neuro 2a cells expressing wt- and mutant HGF/SF proteins were incubated for 2 hours and detected with either the rabbit anti tag antibody HA11 (Hiss diagnostics) or with an anti-HGF/SF mouse monoclonal antibody (clone 34) followed by incubation with HRP-conjugated second antibody. For MET ELISA, a soluble fusion protein consisting of the extracellular domain(s) of MET and the hinge, CH2 and CH3 domain of immunoglobulin γ 1 was produced (GH, unpublished results) and purified by protein A-Sepharose affinity chromatography to ∼ 80% homogeneity. Wells were coated with full length wt-HGF/SF or HP1 overnight at 4°C, blocked, and the MET-Ig fusion protein was added (200 ng/ml) for 2 hours followed by incubation with goat anti-human IgG antibody and HRP-conjugated rabbit anti-goat IgG antibody. For colour development 0.66 mg/ml ortho-phenyl diamine in 50 mM phosphate buffer pH 6.0 was used as HRP substrate (100 µl/well).

### Heparin binding kinetics of full length wt-HGF/SF and HP1 and their NK2 fragments

Kinetic analysis of the binding of wild-type and mutant proteins was carried out by surface plasmon resonance (SPR) using a BIAcore 2000 instrument (BIAcore Ltd., St. Albans, Herts., UK). 10 mg/ml high molecular weight heparin (Sigma H3393) was biotinylated with a three fold excess of NHS-LC-biotin (Pierce 21336) in 1 ml of 100 mM phosphate buffer pH 8.0 for 1 hour at RT. Biotinylated heparin was separated from free biotin derivatives by sequential passage through two 2ml Excellulose columns (Pierce) and used for capture on the surface of streptavidin-coated SA sensor chips. All experiments were carried out at an operating temperature of 25°C. The running buffer (EB) in all runs was PBS containing 0.2mM EDTA, 0.5 g/l NaN_{3.} 0.05 g/l p20 detergent (BIAcore Ltd). In order to prevent possible leakage of heparin from the test surfaces (flowcells 2-4) to the control one (flowcell 1), surface 1 was blocked with two 20 *µ*l injections of 250 ng/ml biotin in EB at a flow-rate of 5 *µ*l/min. Surface 2 was then coated at 5 *µ*l/min with 10 ml of 2 nM heparin-biotin in EB. Approximately 25 RU were bound in this example; since a small increase in signal may occur due to crosslinking of the dextran by the long heparin molecules, an exact estimate of the heparin bound at low levels is not possible. The surface was then blocked with biotin as for surface 1. To prevent absorption or denaturation of the protein samples for SPR analysis, these were routinely diluted in 0.2 mg/ml BSA in 0.2 mM EDTA which had been pre-absorbed onto Heparin-Sepharose4B (Pharmacia Biotech, St. Albans) for 30 min at room temperature. For analysis of the various proteins, samples were pumped at 15 *µ*l/min. Regeneration of the surface was by sequential "QUICKINJECT/EXTRACLEAN" of 10 *µ*l of 0.1 g/l SDS in water followed by 2 x 10 *µ*l of 1M NaCl. Finally 15 ml of 0.2 mg/ml BSA in EB were injected to block any non-specific binding sites. Data files from the automated analysis runs were analysed by non-linear curve fitting (34) using the BIAcore BIAevaluation software (BIAcore Ltd). Protein concentrations were measured by the BCA reagent (Pierce) and the biological activity of each batch of protein confirmed in the MDCK assay (7) to ensure consistent specific activity.

### Cell biological assays

Colony scatter assays of MDCK and mink lung cells were carried out as previously described (7). For measurement of DNA synthesis, mink lung cells were plated at a density of 5,000-10,000 cells/well in 24 well plate and serum-starved by incubation in DMEM for 48 hours. Different concentrations of HGF/SF and HP1 were then added and incubation continued for 24 hours in DMEM containing 100 µg/ml BSA before addition of ³H-thymidine (1 µCi/well) and a final incubation for 16 hours. Radioactivity insoluble in 5 % TCA was measured by scintillation counting after solubilization in 0.2 M NaOH.

### Total binding of wt-HGF/SF and HP1 to mink lung cells

Mink lung cells were grown to confluence in 24 well plates. After two washes with medium, cells were incubated at 4°C for 30 min. Serial dilutions of HGF/SF and HP1 in PBS were then added to the cells and incubated for another 15 min at 4°C. Unbound factor was removed by three washes with PBS and cells fixed with 4% paraformaldehyde in PBS. After two further washes the cells were incubated with a mouse anti HGF/SF monoclonal antibody (clone 34) which reacted in an identical manner with wt-HGF/SF and HP1 followed by incubation with FITC conjugated anti-mouse IgG.

### Clearance and tissue distribution of wt-HGF/SF and HP1 in rats

10 µg of pure HGF/SF and HP1 were iodinated by a solid phase method using Iodogen (Pierce) by Biotez, Berlin, Germany. After the reaction, 1 mg/ml BSA was added and free Iodine was removed by multiple filtration on a Centrisat filtration apparatus (Sartorius) with a 30 kDa cut off. The specific activities were calculated to be 1.85 MBq/mg for HGF/SF and 1.95 MBq/mg for HP1. The biological activities of the proteins before and after iodination was compared in a motility assay using MDCK cells and determined to be 80% after iodination. Adult rats (3 per groups) were anaesthetised, a catheter implanted into the jugular vein and a single iv bolus injection of 0.2 ml of ¹²⁵I-HGF/SF was given (0.45 MBq for wt-HGF/SF and 0.38 MBq for HP1 per animal). After 1, 3, 5, 7, 10 and 60 mins, 2 hours and 20 hours, 200 *µ*l samples of blood were taken *via* the catheter. Blood was allowed to clot and centrifuged at 10,000 g for 5 min at 4°C. For each sample 100 µl were diluted with an equal volume of PBS and 2 ml of ice-cold 10% TCA were added. After 30 min on ice, samples were centrifuged and the precipitate washed with 5% TCA before counting. For measurement of tissue radioactivity, animals were sacrificed 2 hours after injection of ¹²⁵I-labelled wt-HGF/SF or HP1 and perfused with PBS. Organs were taken out, weighted, homogenised and the amount of TCA precipitatable radioactivity determined.

### Effect of wt-HGF/SF and HP1 on DNA synthesis in adult liver

The ability of wild-type HGF/SF and HP1 to induce DNA synthesis in liver cells was measured following intravenous administration of recombinant proteins and cell labelling with BrdU. Briefly, female animals (6-8 weeks old; 19.5 to 26.9 g of body weight) were injected twice daily with either PBS, wild-type HGF/SF or HP1 in the tail vein (55.5 µg/dose) for three days. At the time of the last injection of factor, each animal received BrdU (75 µg/g body weight) was injected intraperitoneally and 12 hours later the animals were sacrificed, the liver was dissected and frozen sections were prepared. Sections were fixed in 70% ethanol (5 mins), treated with 2.4 M HCl for 10 mins at 37°C and nuclei that had incorporated BrdU were stained with anti-BrdU antibody (Sigma, 1:400) followed by peroxidase-conjugated anti-mouse IgG (Jackson Laboratories, 1:500).

### Results

### Hairpin structure and kringle 2 mutants of HGF/SF

The N-terminal domain of HGF/SF contains four cysteine residues that are conserved in the HGF/SF homologues HGF1/MSP and plasminogen. Studies on plasminogen (35) indicated that this domain forms an hairpin structure stabilised by two disulphide bonds. A three dimensional model of the hairpin structure of HGF/SF suggested the presence of several positively charged residues which were absent in the corresponding models of HGF1/MSP and plasminogen (29). Six different point mutants of the hairpin structure of HGF/SF were constructed (HP1 to HP6) in which a total of 10 amino acid residues were substituted. The strategy involved mutation of positive to negative, negative to positive and hydrophobic to polar (see legend to Fig. 1 for the sequence of the mutants). The activity of these point mutants was compared to those of wild type (wt-) HGF/SF and HPΔ, a mutant in which the whole hairpin structure (amino acids 68 to 100) was deleted. In addition, two multiple mutants of kringle 2 were constructed: in mutant Kr2-1 a cluster of four positive residues on the surface of the kringle (H241, R242, K244 and R249) was mutated; in mutant Kr2-2 three residues were mutated (R281, W282 and Y284) that had been predicted to form a hydrophobic pocket on the surface of kringle 2 similar to the one present in plasminogen kringle 4 (29).

For initial studies, mutants were expressed by transient transfection in the Neuro2A cell line (Fig. 1a) and characterized for heparin binding (Fig. 1b) and biological activity (Fig. 1c). As expected HPD, the mutant lacking the hairpin structure, exhibited neither heparin binding nor biological activity. The point mutants fell in three different groups: group 1 (HP1, HP2 and HP4) exhibited normal signalling activity but much decreased (HP1 and HP2) or decreased (HP4) heparin-binding; group 2 (HP3) showed a severe loss in both signalling and heparin-binding and group 3 (HP5 and HP6) behaved essentially like wild-type HGF/SF (Fig. 1b and c). The first kringle 2 mutant, Kr2-1 showed severely reduced heparin-binding and biological activity whereas the second one, Kr2-2, was indistinguishable from wt-HGF/SF (Fig. 1b and c). From these results we can conclude that a first cluster of positively charged residues in the hairpin structure (R73, R76) is involved in heparin but not in MET binding and that residues R281, W282 and Y284 are involved in neither. It also appears that two extra clusters of positively charged residues, one in the hairpin structure (K91 and K94) and one in kringle 2 (H241, R242, K24 and R 249) may participate both in MET and heparin binding.

### Binding kinetics of wt-HGF/SF, HP1 and their NK2 fragments to heparin

Full-length wt-HGF/SF and HP1 (one of the two mutants showing greatly reduced heparin-binding but normal biological activity) were expressed in insect cells in order to analyse their kinetics of binding to heparin by surface plasmon resonance (SPR). These experiments revealed important differences in the binding kinetics of HP1.

The binding to heparin of wt-HGF/SF (4 to 30 nM) revealed a single first-order dissociation rate constant (k_{off}) of ∼ 1 x 10⁻³ s⁻¹. The bulk of the binding during association could be fitted to a single-site model with an association rate constant (kₒₙ) of ∼ 1 x 10⁶ M⁻¹ s⁻¹, giving a molar dissociation constant (K_{d}) of ∼ 1 x 10⁻⁹ M. HP1 (4 to 80 nM) showed much reduced heparin binding (data not shown). However we considered that the difference observed between wt-HGF/SF and HP1 might have been affected by the presence of dimeric or oligomeric forms of the two proteins, a point that could not be established due to low recovery from size-exclusion columns. We therefore expressed the NK2 fragments of wt-HGF/SF and HP1 (ie all the domains shown to be involved in heparin binding, Mizuno et al., 1994) in the methylotropic yeast *P. pastoris* and used these for further kinetic analysis. wt-NK2 and HP1-NK2 purified from the supernatant of *P. pastoris* cultures consisted of a mixture of NK2 monomers and dimers that could be readily separated on Superdex-75 columns, allowing the binding kinetics to heparin to be established with *bona fide* monomeric species.

Sensorgrams overlays of the binding to heparin of wt-NK2 (Fig. 2a) and HP1-NK2 (Fig. 2b) confirmed major differences in heparin binding. For wt-NK2, the dissociation curves were slower at the higher concentrations and apparent k_{off} values varied between 1 and 4 x 10⁻³ s⁻¹ (Fig. 2c, solid squares). In contrast, the dissociation of HP1-NK2 (Fig. 2b) rose with concentration to an apparent k_{off} value of 6-7 x 10⁻³ s⁻¹ (Fig. 2c, open squares). Analysis of the association phase of wt-NK2 (Fig. 2a) showed complex kinetics. Assuming a single class of binding sites and taking data from the earliest (fastest) phase, plots of the observed association rate constant ("Kₛ") *νs* C for concentrations up to 300 nM gave a straight line (r² = 0.997) from which we calculated a kₒₙ of 1.75 x 10⁶ M⁻¹ s⁻¹ (Fig. 2d, full squares). In the later stages of binding a second phase with much slower association was discernible but attempts to fit models based on two classes of binding sites were inadequate. Taking the fastest value for k_{off} of 4 x 10⁻³ s⁻¹ and a value for kₒₙ of 1.75 x 10⁶ M⁻¹ s⁻¹, a K_{d} value of 2.3 nM was calculated for wt-NK2.

For HP1-NK2, analysis of the early phase of binding (assuming the presence of a single class of binding sites) gave a kₒₙ value of ∼ 5 x 10⁴ M⁻¹ s⁻¹ from the Kₛ *vs* C plot (Fig. 2d, open squares). This figure was ∼ 35 fold lower than for wt-NK2. Taking a value of 6 x 10⁻³ s⁻¹ for k_{off} (Fig. 2c, open squares), a K_{d} of 120 nM was calculated for HP1-NK2. The binding affinity of HP1-NK2 is therefore over 50 fold lower than for wt-NK2.

### Binding of wt-HGF/SF and HP1 to HSPGs and MET and activity of the two proteins on target cells in vitro

The binding of wt-HGF/SF and HP1 to cell surface and matrix heparan sulphates was investigated in several cell types, including the carcinoma lines HepG2 and Hep3B and mink lung cells. The latter have recently been shown to bind large amounts of HGF/SF and its truncated forms NK1 and NK2, via HSPGs (36). By immunofluorescence, the total binding of HP1 to HepG2, Hep3B and mink lung cells was much lower than for wt-HGF/SF (data not shown). These results were confirmed and extended by a quantitative solid phase assay and a representative experiment, illustrating the binding of full length wt-HGF/SF and HP1 to mink lung cells, is shown in Fig. 3. The total binding of wt-HGF/SF to mink lung cells increased steadily from 10 to 675 ng/ml whereas binding of HP1 was hardly measurable over the same range of concentrations. Exogenous heparin (0.1 mg/ml) competed effectively for the binding of wt-HGF/SF to the cells and reduced it to levels only slightly higher than those of HP1. This confirms that the bulk of cell-bound wt-HGF/SF was associated with HSPGs and that HP1 failed to bind to such molecules.

In marked contrast with the reduced binding of HP1 to heparin and cell-associated HSPGs, the binding of HP1 to a soluble form of the MET receptor and its ability to induce stimulation of DNA synthesis in mink lung cells were only marginally lower than for wt-HGF/SF (Fig. 4a and 4b respectively). Similar results were obtained with the mouse keratinocyte line MK (data not shown). Equally, the ability of HP1 to induce dissociation and scattering of mink lung cells and MDCK cells were indistinguishable from wt-HGF/SF (compare panels b *vs* c and e *vs* f in Fig. 5). Thus, in spite of a considerable decrease in binding affinity for heparin and HSPGs, the ability of the HP1 mutant to induce DNA synthesis and cell movement on target cells was indistinguishable from wt-HGF/SF.

### In vivo activity of wt-HGF/SF and HP1

We investigated the effect of the HP1 mutation on the behaviour of the factor *in vivo.* For this, wt-HGF/SF and HP1 were labelled with ¹²⁵-I and injected in the jugular vein of adult rats. The concentration of the two proteins in serum and in several organs was then measured in the form of TCA-insoluble radioactivity. Fig. 6a shows the clearance curve for wt-HGF/SF and HP1 from serum. In agreement with previous studies (23, 37, 38), we observed a rapid clearance of wt-HGF/SF from the circulation (>98% over the first 15 minutes). The clearance of HP1 was much slower and, at every time point tested, the concentration of HP1 in plasma was 5-10 fold higher than wt-HGF/SF up to 20 hours after injection (Fig. 6a).

The slower clearance of HP1 from serum was associated with an increased amount of the mutant factor in several organs including liver, kidney, lung, heart, testis and spleen (Fig. 6b). The relative distribution of the HP1 mutant and wt-HGF/SF among different organs was similar but the concentration of HP1 was 2-3 fold higher than wt-HGF/SF (Fig. 6b). The ability of wt-HGF/SF to induce DNA synthesis in adult liver was investigated next after intravenous injection of the two proteins. As expected, in the absence of factors DNA synthesis was very low but it increased considerably after administration of either HGF/SF or HP1 (Fig. 6c). However, stimulation by HP1 was 2.7 fold higher than for wt-HGF/SF (Fig. 6c) in line with the increased level of the factor in serum and liver (Fig. 6a and 6b).

### Discussion

### The heparin and receptor binding sites of HGF/SF

Previous studies had shown that the hairpin structure of the N terminal domain of HGF/SF is important for both heparin (24) and receptor binding (25-28). In this study we show that the heparin and receptor binding sites of HGF/SF can be separated by protein engineering and that mutants forms of HGF/SF with much reduced heparin binding have superior biological activity *in vivo.* Given that both the HP1 and HP2 mutants had much decreased affinity for heparin but biological activities comparable to wt-HGF/SF on target cells in culture, we suggest that the mutations in common (R73E and R76E) are important for heparin binding, a conclusion supported by preliminary characterization of the R73E, R76E double mutant. In contrast, substitution of a second cluster of positively charged residues in the hairpin structure (K91, R93 and K94) or of a separate cluster of positively charged residues in kringle 2 (H241, R242, K244 and R 249), abolished both heparin- binding and HGF/SF signalling in target cells. We note that alanine scanning mutagenesis of the N-terminal domain of HGF/SF (39) failed to identify residues in the hairpin structure essential for MET binding and signalling. This may be due to the fact that multiple substitutions of neighbour residues in the hairpin structure (such as the ones that we have introduced) are required to produce measurable changes. The degree of heparin-binding activity was not reported by Lokker *et al* (39).

### Binding kinetics of wt-HGF/SF and its NK2 fragment to heparin

Our data indicate that full length wt-HGF/SF binds heparin with high affinity (K_{d} = ∼ 1 nM). This figure compares with a value of 0.6 nM from a separate study in which binding of full length wt-HGF/SF to ³⁵S - heparin was measured following separation of bound and free ^{3S}S-heparin by ultrafiltration (23). In order to ensure that the measured kinetics resulted from the interaction of monomeric protein species with heparin (and thus establish accurate kinetics for the HP1 mutant) we relied on monomeric NK2 fragments expressed in *P. pastoris.* The dissociation kinetic of wt-NK2 revealed a significant effect on apparent k_{*off*} values at the highest concentrations of protein suggesting cooperative binding of NK2 to heparin (data not shown). Analysis of the stoichiometry of binding at or near saturation indicates approximately 4 wt-NK2 molecules per 16 kDa heparin. Less certain estimates for wt-HGF/SF on low-heparin surfaces provided a value of 3.5 molecules of HGF/SF per heparin molecule. These values suggest that the heparin-binding site of NK2 and HGF/SF encompasses ∼ 14 monosaccharide units, which is consistent with the findings of Lyon et al. (40) who reported that heparan sulphate fragments of 12-14 units were required for high-affinity binding to HGF/SF.

The molar dissociation constant of full length HGF/SF for heparin (K_{d} = 1nM) compares with a value of 50 nM obtained for the interaction of acidic FGF and heparin under similar experimental conditions (41). The on rates of HGF/SF and FGF are similar (1.75 *vs* 1.0 x 10⁶ M⁻¹ s⁻¹ respectively) and the difference in the affinity is attributable to the off-rate (1 x 10⁻³ s⁻¹ *vs* 6 x 10⁻² s⁻¹ for HGF/SF and FGF). The decreased affinity for heparin of HP1 has been achieved by reducing the on-rate (Fig. 2d), a result in line with the emerging role of electrostatic interactions on association rates (42).

### Different roles for heparan sulphates in the HGF/SF and FGF systems

The HP1 mutant has provided a number of insights on the role of heparin-binding in HGF/SF activity. HGF/SF is one of a number of growth factors that bind HSPGs but the role of heparan sulphate in growth factor activity is generally not clear except in the case of FGF. With FGF there is evidence that three components (ligand, receptor and heparan sulphate) are required for signalling. This conclusion is supported by transfection experiments (2,3) and biochemical studies with purified components (43). HSPGs probably act by dimerising FGF thus promoting receptor dimerization and signalling (44), an interpretation consistent with the recent structures of FGF-heparin complexes (31).

Our results indicate a different role for HSPGs in the HGF/SF system. Although another study suggests that heparin enhances the activity of HGF/SF and the NK1 and NK2 fragments on cells in culture (36), we find that the receptor binding and biological activity of the HP1 mutant are essentially indistinguishable from wt-HGF/SF in spite of a > 50 fold loss in heparin binding.

Our results do, however, raise the question of the physiological function of HGF/SF binding to HSPGs. The *in vivo* data presented here suggest a role for HSPGs in promoting the internalisation and degradation of HGF/SF in tissues (Fig. 6a and 6b). Thus HSPGs may represent a means of clearing HGF/SF in vivo. Indeed there is a striking parallel between the increased DNA synthesis activity of HP1 in liver (Fig. 6c) and the increased level of factor in the tissue (Fig. 6b). It is possible to speculate on other possible roles for HSPGs. For example, HSPGs may help localize HGF/SF to selected cell or tissue compartments, as required for the establishment of a 'morphogen gradient' during embryogenesis (45,46). An example of this process may be the developing limb, where migration of myogenic precursor cells in the bud is known to depend critically on HGF/SF and MET (47).

### Applications of heparin-deficient HGF/SF mutants in vivo

There has been considerable interest recently in potential therapeutic applications of HGF/SF and several studies have addressed the question of the pharmacokinetics of the growth factor *in vivo.* The results of these studies indicated HGF/SF is cleared very rapidly from the circulation (23, 37, 38) and that the half life of the growth factor in plasma could be extended by complexing it with either heparin (23) or other polyanions, such as dextran sulphates (48). HGF/SF infused as a complex with dextran sulphate also had significantly higher activity in inducing hepatocyte proliferation in adult animals (48). Our work demonstrates that equal or superior results can be achieved with engineered forms of HGF/SF, such as the HP1 mutant. The mutant has a longer residence time in the circulation (Fig. 6a), greater tissue levels (Fig. 6b) and is more active than wt-HGF/SF in inducing DNA synthesis in adult liver (Fig. 6c). It therefore overcomes the need to complex HGF/SF with heparin-like compounds in order to improve pharmacokinetics and activity.

Although it is well established that polypeptide growth factors exert their effects on target cells via specific membrane receptors (1), it has become clear in recent years that a number of these proteins also bind cell surface and matrix proteoglycans, especially of the heparan sulphate type (HSPGs). The biological significance of this dual interaction is well understood in the case of fibroblast growth factor (FGF). Cells expressing the FGF receptor but lacking membrane-bound heparan sulphate fail to respond to basic-FGF (2,3) implying heparan sulphate as an essential co-receptor for FGF signalling. Other growth factors, such as the epidermal growth factor (EGF) - related polypeptides amphiregulin (4) and heparin binding - EGF (HB-EGF) (5) as well as several splice variants of vascular endothelial growth factor (VEGF) (6) also require cell-bound heparan sulphate for activity although the role of HSPGs in these systems is less defined.

Hepatocyte growth factor/scatter factor (HGF/SF) (7-12) is a high-molecular weight polypeptide growth factor distinct from the others and with a domain structure related to that of the blood proteinase precursor plasminogen. Like plasminogen, HGF/SF is produced as an inactive, single chain polypeptide which is converted proteolytically into a two-chain species (13-15). Two-chain HGF/SF induces growth, movement and differentiation in target cells *via* a specific membrane tyrosine kinase receptor encoded by the c-MET protooncogene (16,17). HGF/SF binds tightly to the MET receptor with a K_{d} in the sub nanomolar range (18,19). HGF/SF also binds heparin (20,21). This does not appear to affect the binding affinity for a soluble form of the MET receptor although it does increase receptor phosphorylation and mitogenicity on target cells (22). HGF/SF-heparin complexes also exhibit sustained plasma levels and reduced hepatic clearance *in vivo* (23).

The modular structure of HGF/SF has facilitated the identification of the domains responsible for receptor and heparin-binding. Deletion of the hairpin structure of the N-terminal domain (a domain homologous to plasminogen's activation peptide) or kringle 2 results in mutants with reduced apparent affinities for heparin (24). By contrast, deletions of either kringle 1, 3, 4 or the proteinase domain, have little or no effect (24). Thus the heparin binding site(s) of HGF/SF appear to be contain in the hairpin structure of the N terminal domain and kringle 2. The same domains, however, are critical for MET binding and signalling (25-28).

### Example 2: Treatment with variant HGF

Patients with acute liver disease either due to viral infection or chemical/drug injury are treated with the variant HGF by daily iv injections in saline at the dose of 1-100 *µ*g/kg throughout the acute phase of the disease. The development of the disease is monitored by measuring the plasma levels α fetoprotein, γ-globulin, SGOT, SGPT and γ-GT.

### References

1. Ullrich, A. and Schlessinger, J. (1990) *Cell* **61**: 203-212.
2. Yayon, A., Klagsbrun, M, Esko, J.D., Leder, P., and Ornitz D.M. (1991) *Cell* **64**: 841-848.
3. Rapraeger, A.C., Krufka, A., and Olwin, B.B. (1991) *Science* **252**: 1705-1708.
4. Johnson, G.R., and Wong, L. (1994) *J. biol. Chem.* **269**: 27149-27154.
5. Igashiyama, S., Abraham, J.A., and Klagsbrun, M. (1993) *J. Cell Biol.* **122**: 933-940
6. Cohen, T., Gitay-Goren, H., Sharon, R., Shibuya, M., Halaban, R., Levi, B.-Z., and Neufeld, G. (1995) *J. biol. Chem.* **270**: 11322-11326.
7. Stoker, M., Gherardi, E., Perryman, M., and Gray, J. (1987) *Nature* **327**: 239-242.
8. Gherardi, E., Gray, J., Stoker, M., Perryman, M. and Furlong, R. (1989) *Proc. natl. Acad. Sci. U.S.A.* **86**: 5844-5848.
9. Nakamura, T., Nawa, K., Ichihara, A., Kaise, N., and Nishino, T. (1987) *Fed. Eur. biochem. Soc. Lett.* **224**, 311-316.
10. Nakamura, T., Nishizawa, T., Hagiya, M., Seki, T., Shimonishi, M., Sugimura, A., Tashiro, K., and Shimizu, S. (1989) *Nature* **342**: 440-443.
11. Miyazawa, K., Tsubouchi, H., Naka, D., Takahashi, K., Okigaki, M., Arakaki, N., Nakayama, H., Hirono, S., Sakiyama, O., Takahashi, K., Godha, E., Daikuhara, Y., and Kitamura, N. (1989) *Biochem. biophys. Res. Commun.* **163**: 967-973.
12. Weidner, K.M., Arakaki, N., Hartmann, G., Vandekerckhove, J., Weingart, S., Rieder, H., Fonatsch, C., Tsubuochi, H., Hishida, T., Daikuhara, Y., and Birchmeier, W. (1991) *Proc. natl. Acad. Sci. U.S.A.* **88**: 7001-7005.
13. Miyazawa, K., Shiniomura, T., Kitamura, A., Kondo, J., Morimoto, Y., and Kitamura N. (1993) *J. biol. Chem.* **268**: 10024-10028.
14. Naldini, L., Tamagnone, L., Vigna, E., Sachs, M., Hartmann, G., Birchmeier, W., Daikuhara, Y., Tsubouchi, H., Blasi, F., and Comoglio, P. M. (1992) *Eur. Mol. Biol. Org.* J. **11**: 4825-4833.
15. Mars, W. M., Zamegar, R., and Michaiopoulos, G. K. (1993) *Amer. J. Pathol.* **143**: 949-958.
16. Bottaro, D.P., Rubin, J.S., Faletto, D.L., Chan, A.M.-L., Kmiecick, T.E., Vande Woude, G.F., and Aaronson S.A. (1991) *Science* **251**: 802-804.
17. Naldini, L., Weidner, K. M., Vigna, E., Gaudino, G., Bardelli, A., Ponzetto, C., Narsimhan, R. P., Hartmann, G., Zamegar, R., Michalopoulos, G. K., Birchmeier, W. and Comolgio, P. (1991) *Eur. Mol. Biol. Org. J.* **10**: 2867-78.
18. Arakaki, N., Hirono, S., Ishii, T., Kimoto, M., Kawakami, S., Nakayama, H., Tsubouchi, H., Hishida, T. and Daikuhara, Y. (1992) *J. biol. Chem.* **267**: 7101-7107.
19. Komada, M., Miyazawa, K., and Kitamura, N. (1992) *Eur. J. Biochem.* **204**: 857-864.
20. Rosen, E: M., Goldberg, I. D., Kacinski, B. M., Buckholz, T., and Vinter, D. W. (1989) *In Vitro Cell dev. Biol.* **25**: 163-73.
21. Weidner, M. K., Behrens, J., Vandekerckfiove, J. and Birchmeyer, W. (1990) *J. Cell Biol.* **111**: 2097-2108.
22. Zioncheck, T.F., Richardson, L., Liu, J., Chang, L., King, K.L., Bennett, G.L., Fugedi, P., Chamow, S.M., Schwall, R.H., and Stack, R.J. (1995). *J. biol. Chem.* **270**: 16871-16878.
23. Kato, Y., Liu, K.-X., Nakamura, T., and Sugiyama, Y. (1994) *Hepatology* **20**: 417-424.
24. Mizuno, K., Inoue, H., Hagiya, M., Shimizu, S., Nose, T., Shimohigashi, Y., and Nakamura, T. (1994) *J. Biol. Chem.* **269**: 1131-1136.
25. Matsumoto, K., Takehara, T., Inoue, H., Hagiya, M., Shimizu, S., and Nakamura, T. (1991) *Biochem. biophys. Res. Commun.* 181: 691-699.
26. Hartmann, G., Naldini, L., Weidner, K.M., Sachs, M., Vigna, E., Comoglio, P.M. and Birchmeier, W. (1992) *Proc natl. Acad. Sci. U.S.A.* **89:** 11574-11578.
27. Lokker, N. A., Mark, M. R., Luis, E. A., Bennett, G. L., Robbins, K. A., Baker, J. B., and Godowski, P. J. (1992) *Eur. Mol. Biol. Org. J.* **11**: 2503-2510.
28. Okigaki, M., Komada, M. Uehara, Y., Miyazawa, K., and Kitamura, N. (1992) *Biochemistry* **31**: 9555-9561.
29. Donate, L., Gherardi, E., Srinivasan, N., Sowdhamini, R., Aparicio, S. and Blundell, T. (1994) *Protein Sci.* **3**: 2378-2394.
30. Carrell, R.W., Stein, P.E., Fermi, G., and Wardell, M.R. (1994) *Structure* **2**: 257-270.
31. Faham, S., Hileman, R.E., Fromm, J.R., Linhardt, R.J., and Rees, D.C. (1996) *Science* **271**: 1116-1120.
32. Frixen, U., Beherens J., Sachs, M., Ebeile, G., Voss, B., Warda, A., Lochner, D. and Birchmeier, W. (1991). *J Cell Biol.* **113**: 173-185.
33. Scorer, C. A., Clare, J. J., McCombie, W. R., Romanos, M. A., & Sreekrishna, K. (1994). *Bio*/*Technology* **12**: 181-184.
34. O' Shannessy, D., J., Brigham-Burke, M., Soneson, K.K., Hensley, P., and Brooks, I. (1994). *Meth. Enzymol.* **240**: 323-349.
35. Wiman, B. (1973) *Eur. J. Biochem.* **39**: 1-9.
36. Schwall, R.H., Chang, L.Y., Godowski, P.J., Kahn, D.W., Hillan, K.J., Bauer, K.D. and Zioncheck, T.F. (1996) *J. Cell Biol.* **133**: 709-718.
37. Appasamy, R., Tanabe, M., Murase, N., Zarnegar, R., Venkataramanan, R., Vanthiel, D.H., and Michalopoulos, G.K. (1993) *Lab. Invest.* **68**: 270-276.
38. Zioncheck, T. F., Richardson, DeGuzman, G.G., Modi, N.B., Hansen, S.E., and Godowski, P.J. (1994). *Endocrinology* **134**: 1879-1887.
39. Lokker, N. A., Presta, L.G. and Godowski, P.J. (1994) *Protein Eng.* **7:** 895-903.
40. Lyon, M., Deakin, J. A., Mizuno, K., Nakamura, T., Gallagher, J.T. (1994) *J. Biol. Chem.* **269**: 11216-11223.
41. Mach, H., Volkin, D.B. Burke, C.J., Middaugh, C.R., Linhardt, R.J., Fromm, J.R., Loganathan, D., and Mattson, L. (1993) *Biochemistry* **32**: 5480-5489.
42. Schreiber, G. and Fersht A.R. (1996) *Nature Struct. Biol.* **3**: 427-431.
43. Pantoliano, M.W., Horlick, R.A., Springer, B.A., van Dyk, D.E., Tobery, T., Wetmore, D.R., Lear, J.D., Nahapetian, A.T., Bradley, J.D., and Sisk, W.P. (1994) *Biochemistry* **33**: 10229-10248.
44. Spivak-Kroizman, T., Lemmon, M.A., Dikic, I., Ladbury, J.E., Pinchasi, D., Huang, J., Jaye, M., Crumley, G., Schlessinger, J., and Lax, I. (1994) *Cell* **79**: 1015-1024.
45. Wolpert, L. (1989) *Development* (Supplement to vol **107**: 3-12).
46. Gurdon, J.B., Mitchell, A. and Mahony, D. (1995) *Nature* **376**: 520-521.
47. Bladt, F. Riethmacher, D. Isenmann, S. Aguzzi, A., and Birchmeier, C. (1195) *Nature* **376**: 768-771.
48. Roos, F., Ryan, A.M., Chamow, S.M., Bennett, G.L., and Schwall, R.H. (1995). *Amer. J. Physiol.* **268**: G380-G386.

## Claims

1. A variant hepatocyte growth factor (HGF) which is substantially incapable of binding a heparan sulphate proteoglycan but which is capable of binding to the HGF receptor wherein a positively-charged amino acid residue in the hairpin loop structure of wild-type HGF has been replaced with an amino acid residue with a negative charge for use in medicine.

2. A variant human hepatocyte growth factor (HGF) according to Claim 1 wherein at least amino acid residue R73 has been replaced by an amino acid residue with a negative charge for use in medicine.

3. A variant human hepatocyte growth factor (HGF) according to Claim 1 or 2 wherein at least amino acid residue R76 has been replaced by an amino acid residue with a negative charge for use in medicine.

4. A variant human hepatocyte growth factor (HGF) according to any one of the preceding claims wherein both amino acid residues R73 and R76 have been replaced independently with an amino acid residue with a negative charge for use in medicine.

5. A variant human hepatocyte growth factor (HGF) comprising amino acid residue replacements R73E and R76E for use in medicine.

6. A variant human hepatocyte growth factor (HGF) comprising amino acid residue replacements R73E, R76E and R93E for use in medicine.

7. A variant human hepatocyte growth factor (HGF) comprising amino acid residue replacements R73E, R76E and K78E for use in medicine.

8. A variant human hepatocyte growth factor (HGF) consisting of human HGF with amino acid replacements R73E and R76E for use in medicine.

9. A variant human hepatocyte growth factor (HGF) consisting of human HGF with amino acid replacements R73E, R76E and R93E for use in medicine.

10. A variant human hepatocyte growth factor (HGF) consisting of human HGF with amino acid replacements R73E, R76E and K78E for use in medicine.

11. A variant hepatocyte growth factor (HGF) according to any one of Claims 1 to 10 which antagonises the action of wild-type HGF for use in medicine.

12. A variant hepatocyte growth factor (HGF) according to Claim 11
wherein the variant HGF further comprises a mutation which confers resistance in the variant HGF to proteolytic cleavage by enzymes capable of *in vivo* conversion of HGF into its two-chain form for use in medicine.

13. A variant human hepatocyte growth factor (HGF) according to Claim 12 which have an amino acid alteration at or adjacent to any of amino acids 493, 494, 495 and 496 of the wild-type human HGF.

14. A pharmaceutical composition comprising a variant hepatocyte growth factor (HGF) as defined in any one of the preceding claims or claims 17 to 24 and a pharmaceutically acceptable carrier.

15. Use of a variant hepatocyte growth factor (HGF) as defined in any one of Claims 1 to 13 in the manufacture of a medicament for treating a patient in need of treatment with a HGF or an antagonist , thereof.

16. Use as defined in Claim 15 wherein the patient has cancer.

17. A variant hepatocyte growth factor (HGF) wherein a positively-charged amino acid residue in the hairpin loop structure of wild-type HGF has been replaced with an amino acid residue with a negative charge provided that the variant HGF is not a variant of human HGF in which the replacements (a) R73E. R76E and R93E or (b) R73E and R76E or (c) K91E, R93E and K94E have been made.

18. A variant human hepatocyte growth factor (HGF) according to Claim 17 wherein at least amino acid residue R73 has been replaced by an amino acid residue with a negative charge.

19. A variant human hepatocyte growth factor (HGF) according to Claim 17 wherein at least amino acid residue R76 has been replaced by an amino acid residue with a negative charge.

20. A variant human hepatocyte growth factor (HGF) according to any one of Claims 17 to 19 wherein both amino acid residues R73 and R76 have been replaced independently with an amino acid residue with a negative charge.

21. A variant human hepatocyte growth factor (HGF) comprising amino acid residue replacements R73E, R76E and K78E.

22. A variant hepatocyte growth factor (HGF) according to Claim 17 which antagonises the action of wild-type HGF.

23. A variant hepatocyte growth factor (HGF) according to Claim 22 wherein the variant HGF further comprises a mutation which confers resistance in the variant HGF to proteolytic cleavage by enzymes capable of *in vivo* conversion of HGF into its two chain form.

24. A variant human hepatocyte growth factor (HGF) according to Claim 23 which have an amino acid alteration at or adjacent to any of amino acids 493, 494, 495 and 496 of the wild-type human HGF.

25. A polynucleotide encoding a variant hepatocyte growth factor according to any one of Claims 17 to 22.

26. A vector comprising a polynucleotide according to Claim 25.

27. A host cell comprising a polynucleotide or vector according to Claim 25 or 26.

28. A method of producing a variant hepatocyte growth factor (HGF) the method comprising culturing a cell as defined in Claim 27 and isolating the variant HGF therefrom.

29. A method of inducing DNA synthesis in a cell in *vitro* comprising providing to the cell a variant hepatocyte growth factor (HGF) as defined in any one of Claims 1 to 10.

30. A method of inducing dissociation and scattering of cells in a cell population *in vitro,* the method comprising providing to the cell population a variant hepatocyte growth factor (HGF) as defined in any one of Claims 1 to 10.

## Patentansprüche

1. Variante des Leberzellenwachstumsfaktors (Hepatocyte Growth Factor = HGF), die im Wesentlichen nicht in der Lage ist, ein Schwefelleberproteoglykan zu binden, die aber in der Lage ist, an den HGF-Rezeptor zu binden, wobei ein positiv geladener Aminosäurerest in der Haarnadelschleifenstruktur des HGF vom Wildtyp durch einen Aminosäurerest mit einer negativen Ladung ersetzt worden ist, zur Verwendung in der Medizin.

2. Variante des humanen Leberzellenwachstumsfaktors (HGF) nach Anspruch 1, wobei mindestens der Aminosäurerest R73 durch einen Aminosäurerest mit einer negativen Ladung ersetzt worden ist, zur Verwendung in der Medizin.

3. Variante des humanen Leberzellenwachstumsfaktors (HGF) nach Anspruch 1 oder 2, wobei mindestens der Aminosäurerest R76 durch einen Aminosäurerest mit einer negativen Ladung ersetzt worden ist, zur Verwendung in der Medizin.

4. Variante des humanen Leberzellenwachstumsfaktors (HGF) nach einem der vorangehenden Ansprüche, wobei beide Aminosäurereste R73 und R76 unabhängig voneinander durch einen Aminosäurerest mit einer negativen Ladung ersetzt worden sind, zur Verwendung in der Medizin.

5. Variante des humanen Leberzellenwachstumsfaktors (HGF), die Aminosäurerestersetzungen R73E und R76 E aufweist, zur Verwendung in der Medizin.

6. Variante des humanen Leberzellenwachstumsfaktors (HGF), die Aminosäurerestersetzungen R73E, R76E und R93E aufweist, zur Verwendung in der Medizin.

7. Variante des humanen Leberzellenwachstumsfaktors (HGF) die Aminosäurerestersetzungen R73E, R76E und K78E aufweist, zur Verwendung in der Medizin.

8. Variante des humanen Leberzellenwachstumsfaktors (HGF) bestehend aus humanem HGF mit Aminosäureersetzungen R73E und R76E, zur Verwendung in der Medizin.

9. Variante des humanen Leberzellenwachstumsfaktors (HGF) bestehend aus humanem HGF mit Aminosäureersetzungen R73E, R76E und R93E, zur Verwendung in der Medizin.

10. Variante des humanen Leberzellenwachstumsfaktors (HGF) bestehend aus humanem HGF mit Aminosäureersetzungen R73E, R76E und K78E, zur Verwendung in der Medizin.

11. Variante des Leberzellenwachstumsfaktors (HGF) nach einem der Ansprüche 1 bis 10, die der Wirkung von HGF vom Wildtyp entgegenwirkt, zur Verwendung in der Medizin.

12. Variante des Leberzellenwachstumsfaktors (HGF) gemäß Anspruch 11, wobei die Variante des HGF weiterhin eine Mutation aufweist, die bei der Variante des HGF zur Beständigkeit gegenüber proteolytischer Spaltung mit Enzymen führt, die zur *in vivo*-Umwandlung von HGF in seine zweikettige Form in der Lage sind, zur Verwendung in der Medizin.

13. Variante des humanen Leberzellenwachstumsfaktors (HGF) nach Anspruch 12, die eine Aminosäureänderung bei oder benachbart irgendeiner der Aminosäuren 493, 494, 495 und 496 des humanen HGF vom Wildtyp aufweist.

14. Pharmazeutische Zusammensetzung mit einer Variante des Leberzellenwachstumsfaktors (HGF), wie er in einem der vorangehenden Ansprüche oder der Ansprüche 17 bis 24 beschrieben ist, und mit einem pharmazeutisch akzeptablen Träger.

15. Verwendung einer Variante des Leberzellenwachstumsfaktors (HGF), wie er in einem der Ansprüche 1 bis 13 beschrieben ist, bei der Herstellung eines Medikaments zur Behandlung eines Patienten, der der Behandlung mit einem HGF oder einem Antagonisten davon bedarf.

16. Verwendung nach Anspruch 15, wobei der Patient Krebs hat.

17. Variante des Leberzellenwachstumsfaktors (HGF), wobei ein positiv geladener Aminosäurerest in der Haarnadelschleifenstruktur des HGF vom Wildtyp durch einen Aminosäurerest mit negativer Ladung ersetzt worden ist, unter der Voraussetzung, dass die Variante des HGF keine Variante des humanen HGF ist, bei der die Ersetzungen (a) R73E, R76E und R93E oder (b) R73E und R76E oder (c) K91 E, R93E und K94E vorgenommen worden sind.

18. Variante des humanen Leberzellenwachstumsfaktors (HGF) nach Anspruch 17, wobei mindestens der Aminosäurerest R73 durch einen Aminosäurerest mit einer negativen Ladung ersetzt worden ist.

19. Variante des humanen Leberzellenwachstumsfaktors (HGF) nach Anspruch 17, wobei mindestens der Aminosäurerest R76 durch einen Aminosäurerest mit einer negativen Ladung ersetzt worden ist.

20. Variante des humanen Leberzellenwachstumsfaktors (HGF) gemäß einem der Ansprüche 17 bis 19, wobei beide Aminosäurereste R73 und R76 unabhängig voneinander durch einen Aminosäurerest mit einer negativen Ladung ersetzt worden sind.

21. Variante des humanen Leberzellenwachstumsfaktors (HGF) mit Aminosäurerestersetzungen R73E, R76E und K78E

22. Variante des Leberzellenwachstumsfaktors (HGF) nach Anspruch 17, die der Wirkung des HGF vom Wildtyp entgegenwirkt.

23. Variante des Leberzellenwachstumsfaktors (HGF) nach Anspruch 22, wobei die Variante des HGF weiterhin eine Mutation aufweist, die bei Variante des HGF zu Beständigkeit gegenüber proteolytischer Spaltung durch Enzyme führt, die zu einer *in vivo*-Umwandlung von HGF in seine zweikettige Form in der Lage sind.

24. Variante des humanen Leberzellenwachstumsfaktors (HGF) nach Anspruch 23, die eine Aminosäureänderung an oder benachbart irgendeiner der Aminosäuren 493, 494, 495 und 496 des humanen HGF von Wildtyp aufweist.

25. Polynukleotid, das eine Variante des Leberzellenwachstumsfaktors nach einem der Ansprüche 17 bis 22 kodiert.

26. Vektor, der ein Polynukleotid nach Anspruch 25 aufweist.

27. Wirtszelle, die ein Polynukleotide oder einen Vector nach Anspruch 25 bzw. 26 aufweist.

28. Verfahren zum Herstellen einer Variante des Leberzellenwachstumsfaktors (HGF), wobei das Verfahren das Kultivieren einer Zelle, wie sie im Anspruch 27 definiert ist, und das Isolieren der Variante des HGF davon aufweist.

29. Verfahren des Einleitens der DNA-Synthese in einer Zelle *in vitro,* das das Bereitstellen einer Variante des Leberzellenwachstumsfaktors (HGF), wie sie in einem der Ansprüche 1 bis 10 definiert ist, für die Zelle aufweist.

30. Verfahren des Einleitens der Aufspaltung und des Ausstreuens von Zellen bei einer Zellpopulation *in vitro,* wobei das Verfahren das Bereitstellen einer Variante des Leberzellenwachstumsfaktors (HGF), wie sie in einem der Ansprüche 1 bis 10 definiert ist, für die Zellpopulation aufweist.

## Revendications

1. Variante du facteur de croissance des hépatocytes (HGF) qui est essentiellement incapable de se lier à un protéoglycane sulfate d'héparane mais qui est capable de se lier au récepteur de l'HGF, dans laquelle un résidu aminoacide chargé positivement dans la structure en épingle à cheveux de l'HGF de type sauvage a été remplacé par un résidu aminoacide ayant une charge négative pour une utilisation en médecine.

2. Variante du facteur de croissance des hépatocytes (HGF) humains selon la revendication 1, dans laquelle au moins le résidu aminoacide R73 a été remplacé par un résidu aminoacide ayant une charge négative pour une utilisation en médecine.

3. Variante du facteur de croissance des hépatocytes (HGF) humains selon la revendication 1 ou 2, dans laquelle au moins le résidu aminoacide R76 a été remplacé par un résidu aminoacide ayant une charge négative pour une utilisation en médecine.

4. Variante du facteur de croissance des hépatocytes (HGF) humains selon l'une quelconque des revendications précédentes, dans laquelle les deux résidus aminoacides R73 et R76 ont été remplacés indépendamment l'un de l'autre par un résidu aminoacide ayant une charge négative pour une utilisation en médecine.

5. Variante du facteur de croissance des hépatocytes (HGF) humains comprenant les remplacements des résidus aminoacides R73E et R76E pour une utilisation en médecine.

6. Variante du facteur de croissance des hépatocytes (HGF) humains comprenant les remplacements des résidus aminoacides R73E, R76E et R93E pour une utilisation en médecine.

7. Variante du facteur de croissance des hépatocytes (HGF) humains comprenant les remplacements des résidus aminoacides R73E, R76E et K78E pour une utilisation en médecine.

8. Variante du facteur de croissance des hépatocytes (HGF) humains composée d'un HGF humain comprenant les remplacements des aminoacides R73E et R76E pour une utilisation en médecine.

9. Variante du facteur de croissance des hépatocytes (HGF) humains composée d'un HGF humain comprenant les remplacements des aminoacides R73E, R76E et R93E pour une utilisation en médecine.

10. Variante du facteur de croissance des hépatocytes (HGF) humains composée d'un HGF humain comprenant les remplacements des aminoacides R73E, R76E et K78E pour une utilisation en médecine.

11. Variante du facteur de croissance des hépatocytes (HGF) selon l'une quelconque des revendications 1 à 10, qui antagonise l'action de l'HGF de type sauvage pour une utilisation en médecine.

12. Variante du facteur de croissance des hépatocytes (HGF) selon la revendication 11, dans laquelle la variante de l'HGF comprend de plus une mutation qui confère une résistance, dans la variante de l'HGF, au clivage protéolytique par des enzymes capables de convertir *in vivo* l'HGF en sa forme à deux chaînes pour une utilisation en médecine.

13. Variante du facteur de croissance des hépatocytes (HGF) humains selon la revendication 12, qui possède une modification des aminoacides au niveau de l'un quelconque des aminoacides 493, 494, 495 et 496 de l'HGF humain de type sauvage ou adjacente à ceux-ci.

14. Composition pharmaceutique comprenant une variante du facteur de croissance des hépatocytes (HGF) telle que définie dans l'une quelconque des revendications précédentes ou des revendications 17 à 24 et un véhicule acceptable sur le plan pharmaceutique.

15. Utilisation d'une variante du facteur de croissance des hépatocytes (HGF) telle que définie dans l'une quelconque des revendications 1 à 13, dans la fabrication d'un médicament pour traiter un patient nécessitant un traitement par un HGF ou un antagoniste de celui-ci.

16. Utilisation telle que définie dans la revendication 15, dans laquelle le patient souffre d'un cancer.

17. Variante du facteur de croissance des hépatocytes (HGF), dans laquelle un résidu aminoacide chargé positivement dans la structure en épingle à cheveux de l'HGF de type sauvage a été remplacé par un résidu aminoacide ayant une charge négative à condition que la variante de l'HGF ne soit pas une variante de l'HGF humain dans laquelle les remplacements (a) R73E, R76E et R93E ou (b) R73E et R76E ou (c) K91E, R93E et K94E ont été effectués.

18. Variante du facteur de croissance des hépatocytes (HGF) humains selon la revendication 17, dans laquelle au moins le résidu aminoacide R73 a été remplacé par un résidu aminoacide ayant une charge négative.

19. Variante du facteur de croissance des hépatocytes (HGF) humains selon la revendication 17, dans laquelle au moins le résidu aminoacide R76 a été remplacé par un résidu aminoacide ayant une charge négative.

20. Variante du facteur de croissance des hépatocytes (HGF) humains selon l'une quelconque des revendications 17 à 19, dans laquelle les deux résidus aminoacides R73 et R76 ont été remplacés indépendamment l'un de l'autre par un résidu aminoacide ayant une charge négative.

21. Variante du facteur de croissance des hépatocytes (HGF) humains comprenant les remplacements des résidus aminoacides R73E, R76E et K78E.

22. Variante du facteur de croissance des hépatocytes (HGF) humains selon la revendication 17, qui antagonise l'action de l'HGF de type sauvage.

23. Variante du facteur de croissance des hépatocytes (HGF) humains selon la revendication 22, dans laquelle la variante de l'HGF comprend de plus une mutation qui confère une résistance, dans la variante de l'HGF, au clivage protéolytique par des enzymes capables de convertir *in vivo* l'HGF en sa forme à deux chaînes.

24. Variante du facteur de croissance des hépatocytes (HGF) humains selon la revendication 23, qui présente une modification des aminoacides au niveau de l'un quelconque des aminoacides 493, 494, 495 et 496 de l'HGF humain de type sauvage ou adjacente à ceux-ci.

25. Polynucléotide codant une variante du facteur de croissance des hépatocytes selon l'une quelconque des revendications 17 à 22.

26. Vecteur comprenant un polynucléotide selon la revendication 25.

27. Cellule hôte comprenant un polynucléotide ou un vecteur selon la revendication 25 ou 26.

28. Procédé pour produire une variante du facteur de croissance des hépatocytes (HGF), le procédé comprenant la culture d'une cellule telle que définie dans la revendication 27 et l'isolement de la variante de l'HGF de celle-ci.

29. Procédé pour induire *in vitro* une synthèse d'ADN dans une cellule comprenant la fourniture à la cellule d'une variante du facteur de croissance des hépatocytes (HGF) telle que définie dans l'une quelconque des revendications 1 à 10.

30. Procédé pour induire la dissociation et la diffusion *in vitro* de cellules dans une population de cellules, le procédé comprenant la fourniture à la population de cellules d'une variante du facteur de croissance des hépatocytes (HGF) telle que définie dans l'une quelconque des revendications 1 à 10.
